# EUROPEAN PATENT APPLICATION

(11) **EP 4 566 618 A1**
(43) Date of publication of application: **11.06.2025**
(21) Application number: 23214497.2
(22) Date of filing: 06.12.2023
(51) Int. Cl.: A61K 38/47, A61K 9/127, A61K 31/137, A61K 31/365, A61K 31/485, A61K 31/7048, A61K 31/7105, A61K 38/26, A61K 47/30, A61K 48/00, A61P 3/04, A61P 13/12, A61P 35/00, C12N 15/52

(54) **NUCLEIC ACID ENCODING KLOTHO FOR USE IN THERAPEUTIC AND NON-THERAPEUTIC METHODS FOR REDUCING BODY WEIGHT**

(71) Applicant: ADvantage Therapeutics, Inc., Miami, FL 33127 (US)
(72) Inventor: ABRAHAM, Menachem, Boston, MA 02116-5451 (US); ABRAHAM, Carmela, Boston, MA 02116-5451 (US)
(74) Representative: SONN Patentanwälte GmbH & Co KG

(57) **Abstract**

The present disclosure relates to therapeutic and non-therapeutic methods for obtaining or promoting weight loss in a mammal, especially in a human individual, comprising supplementing a cell of the mammal with a Klotho protein by administering to the cell a nucleic acid encoding the Klotho protein such that the nucleic acid is expressed within the cell to produce the Klotho protein so as to obtain weight loss in said mammal.

## Description

### Field of the disclosure

The present disclosure relates to compositions and methods for increasing the level of Klotho protein in a cell or in a subject and in particular to compositions and methods for treating diseases or conditions associated with Klotho deficiency. The methods described herein involve supplementing the level of Klotho protein in a cell by administering to the cell a nucleic acid encoding the Klotho protein.

### Background of the disclosure

Any discussion of the prior art throughout the specification should in no way be considered as an admission that such prior art is widely known or forms part of the common general knowledge in the field.

The single copy gene Klotho plays important roles in ageing, cognition, anti-oxidative stress, neurological protection and development, muscle function, cardiovascular function, kidney health and cancer. Klotho is a Type I transmembrane protein which is mainly expressed in the brain, kidney and reproductive organs (Masuda et al., 2005. Mech. Ageing Dev. 126(21): 1274-1283). The human Klotho gene encodes two transcripts, one generating a ~135kDa transmembrane protein (m-KL), and an alternative splice variant that generates a ~70-kDa secreted protein (s-KL). The m-KL isoform contains an N-terminal signal sequence, an extracellular domain with two internal repeats (KL1 and KL2, each of which is about 550 amino acids in length), a single transmembrane domain and a short intracellular domain. The alternative splice variant includes the KL1 domain and a 15-amino acid tail that is not found in the m-KL transcript. The extracellular domain of the transmembrane form can be cleaved by metalloproteinases ADAM10 and ADAM17, which results in another form of soluble Klotho of about 130 kDa (sometimes referred to as proteolyzed Klotho, or p-KL), which has been detected in serum, urine and cerebrospinal fluid. Studies have also indicated that there is a second recognition site for these proteases located between the KL1 and KL2 domains, which generates two 70-kDa isoforms, one containing the KL1 domain only (similar to the s-KL isoform generate from alternative splicing, but without the 15-amino acid tail), and the other one containing the KL2 domain. Both the transmembrane and soluble forms of Klotho have important functions in many homeostatic processes.

Klotho promotes oligodendrocyte maturation, and it protects neurons from oxidative stress by increasing expression of antioxidant factors. It also induces re-myelination *in vivo* in the cuprizone-induced demyelination model of multiple sclerosis (Zeldich et al., 2015. J. Mol. Neurosci. 57(2): 185-196). Studies have shown that Klotho overexpression reduces cognitive deficits in a mouse model of Alzheimer's disease, and that it enhances cognition in humans and mice (Dubal et al., 2014. Cell Rep. 7(4): 1065-1076; Dubal et al., 2015. Off. J. Soc. Neuroscience. 35(6): 2358-2371). Increased Klotho protein level has also been shown to have a therapeutic effect in chronic kidney disease, acute kidney injury, muscle disorders, cardiovascular disease and cancer.

### Summary of the disclosure

In one aspect, the present disclosure provides a non-therapeutic method ("cosmetic method") for obtaining or promoting weight loss in a mammal, especially in a human individual, comprising supplementing a cell of the mammal with a Klotho protein by administering to the cell a nucleic acid encoding the Klotho protein such that the nucleic acid is expressed within the cell to produce the Klotho protein so as to obtain weight loss in said mammal.

In another aspect, the present disclosure provides a method of treating a mammal, especially a human individual, with pathologically increased body weight, the method comprising administering to the subject a therapeutically effective amount of a nucleic acid encoding a Klotho protein such that the nucleic acid is expressed within a cell of the subject to produce the Klotho protein in an efficient amount to obtain weight loss in said mammal. This aspect is therefore also drawn to a nucleic acid encoding a Klotho protein for use for reducing body weight.

In some examples, the cell is a liver cell, kidney cell, brain cell or a muscle cell.

The treatment may be performed on healthy individuals as non-therapeutic weight loss treatment. The treatment may also be performed on individuals suffering from increased body weight to obtain or promote a therapeutic weight loss treatment. Diseases connected with increased body weight or wherein increased body weight is an additional and severe risk factor are well-known in the present field; it is therefore possible to clearly distinguish individuals which are in need of a therapeutic weight loss treatment and individuals for which a loss of body weight treatment is non-therapeutical, i.e. cosmetic; for example due to lifestyle reasons. Treatment of individuals suffering from obesity is therefore always a therapeutic treatment for the present invention whereas the treatment of healthy individuals with normal body weight is always non-therapeutical. In some embodiments, the treated individual has BMI of at least 25, or between 25 and 29.9. In some embodiments, the treated individual has BMI of at least 30. In some embodiments, the treated individual has BMI of at least 27 and a weight-related disease or condition.

In some examples, the nucleic acid is an RNA. In some embodiments, the nucleic acid is an mRNA. In some embodiments, the nucleic acid is in a viral or non-viral vector.

Preferably, the nucleic acid is administered in a lipid nanoparticle. In some embodiments, the nucleic acid is an mRNA in a lipid nanoparticle.

The nucleic acid may be administered intravenously, intramuscularly, intranasally or intraperitoneally. In some examples, the nucleic acid is administered at least once per week for at least 2 weeks. In some examples, the nucleic acid is administered at least twice per week for at least 2 weeks. In some examples, the nucleic acid is administered at least twice per week for more than 2 weeks.

The nucleic acid may encode a transmembrane isoform of Klotho (m-KL) between about 130 kDa and 140 kDa. The nucleic acid may encode a soluble KL1+KL2 isoform of Klotho between about 125 kDa and 135 kDa. The nucleic acid may encode a secreted isoform of Klotho (s-KL) between about 65 kDa and 75 kDa or a KL1 isoform between about 65 kDa and 75 kDa. In some examples, the Klotho is a KL-VS variant. In some embodiments, the isoform is an isoform of human Klotho protein. In some embodiments, the nucleic acid is an mRNA (e.g., human mRNA) encoding an isoform of human Klotho protein. In some embodiments, the nucleic acid is codon optimized for human expression (e.g., a mouse mRNA that has been codon optimized for human expression).

In some examples, the nucleic acid does not integrate into the genome of the cell.

In some examples, the nucleic acid is administered in combination with an agent that increases endogenous Klotho expression. Suitable agents may include a dietary supplement such as Epigallocatechin Gallate, Oeluropein, Rhein/Rhubarb, Sulphoraphane, Ampelopsin, Astaxanthin, Astragaloside, Astragalus, Cholecalciferol, Curcumin, Quercetin, Genistein or Ginseng. Suitable agents may also include compounds such as Atorvastatin, Cerivastatin, Fluvastatin, Pitavastatin, Rosuvastatin, Simvastatin, Losartan, Valsartan, Resveratrol, Paricalcitrol, Pentoxifylline, Hydrochlorothiazide, Berberine, Dapagliflozin, Dasatinib, Docosahexaenoic acid, Doxorubicin, Empagliflozin, Eplerenone, Everolimus, Cholecalciferol (Vitamin D), Colchicine, Chlorothiazide, Calcitriol, Canagliflozin, Captorpil, Azacitidine, Decitabine, Dihydroartemisinin, Adrenomedullin, Alfacalcidol, Metmorfin, Rosiglitazone, Spironolactone or Udenafil.

### Brief description of drawings

**Figure** 1: Encapsulation percentage of LNPs. As shown by the drop in percentage from 100 to 33 for m-KL and from 100 to 36 for soluble KL, addition of 2.5% Triton promoted release of RNA from the LNPs.
**Figure 2****:** Tapestation analysis of mRNA after *in vitro* transcription (IVT).
**Figure 3****:** Klotho protein production in HEK293T cells after transfection with Klotho mRNA LNPs.
**Figure 4****:** ELISA standard curve for quantifying Klotho in mouse serum.
**Figure 5****:** ELISA results comparing C57 mice treated with LNP-Klotho (Formulation 1) vs untreated mice.
**Figure 6****:** One-way ANOVA comparing ELISA results.
**Figure 7****:** ELISA results comparing C57 mice treated with LNP-Klotho [Formulation 2, Formulation 3 and Formulation 3 without Klotho (empty)].
**Figure 8****:** Reduction of body weight in mice administered Klotho RNA LNPs, empty LNPs or no treatment controls.

### Detailed description

### Definitions

In the context of this specification, the terms "a" and "an" are used herein to refer to one or to more than one (ie, to at least one) of the grammatical object of the article. By way of example, "an element" means one element or more than one element.

The term "about" is understood to refer to a range of +/- 10%, preferably +/- 5% or +/-1% or, more preferably, +/- 0.1%.

The terms "administration concurrently" or "administering concurrently" or "coadministering" and the like refer to the administration of a single composition containing two or more actives, or the administration of each active as separate compositions and/or delivered by separate routes either contemporaneously or simultaneously or sequentially within a short enough period of time that the effective result is equivalent to that obtained when all such actives are administered as a single composition.

By "simultaneously" is meant that the active agents are administered at substantially the same time, and preferably together in the same formulation.

The terms "comprise", "comprises", "comprised" or "comprising", "including" or "having" and the like in the present specification and claims are used in an inclusive sense, *ie,* to specify the presence of the stated features but not preclude the presence of additional or further features.

The term "identity" refers to a relationship between the sequences of two or more polypeptide molecules or two or more nucleic acid molecules, as determined by aligning and comparing the sequences.

The percent identity between two sequences is a function of the number of identical positions shared by the sequences when the sequences are optimally aligned (ie, % homology = # of identical positions/total # of positions x 100), with optimal alignment determined taking into account the number of gaps, and the length of each gap, which need to be introduced for optimal alignment of the two sequences. The comparison of sequences and determination of percent identity between two sequences can be accomplished using a mathematical algorithm.

The percent identity between two nucleotide sequences can be determined using the GAP program in the GCG software package, using a NWSgapdna.CMP matrix and a gap weight of 40, 50, 60, 70, or 80 and a length weight of 1, 2, 3, 4, 5, or 6. The percent identity between two nucleotide or amino acid sequences can also be determined using the algorithm of E. Meyers and W. Miller (CABIOS, 4: 11-17 (1989)) which has been incorporated into the ALIGN program, using a PAM120 weight residue table, a gap length penalty of 12 and a gap penalty of 4. In addition, the percent identity between two amino acid sequences can be determined using the Needleman and Wunsch (J. Mol. Biol. (48):444-453 (1970)) algorithm which has been incorporated into the GAP program in the GCG software package, using either a Blossum 62 matrix or a PAM250 matrix, and a gap weight of 16, 14, 12, 10, 8, 6, or 4 and a length weight of 1, 2, 3, 4, 5, or 6.

The term "isolated" as used herein refers to material that is substantially or essentially free from components that normally accompany it in its native state. For example, an "isolated polynucleotide" as used herein refers to a polynucleotide which has been purified from the sequences which flank it in a naturally-occurring state, eg, a DNA fragment which has been removed from the sequences that are normally adjacent to the fragment. Alternatively, an "isolated peptide" or an "isolated polypeptide" and the like, as used herein, refer to *in vitro* isolation and/or purification of a peptide or polypeptide molecule from its natural cellular environment, and from association with other components of the cell, ie, it is not associated with *in vivo* substances.

The term "operably connected" or "operably linked" as used herein refers to the functional relationship between two or more nucleic acid segments such as a gene and a regulatory element including but not limited to a promoter, which then regulates the expression of the gene.

The term "pharmaceutically acceptable" as used herein refers to substances that do not cause substantial adverse allergic or immunological reactions when administered to a subject. A "pharmaceutically acceptable carrier" includes, but is not limited to, solvents, coatings, dispersion agents, wetting agents, isotonic and absorption delaying agents and disintegrants.

The term "polynucleotide variant" refers to polynucleotides displaying substantial sequence identity with a reference polynucleotide sequence or polynucleotides that hybridize with a reference sequence under stringent conditions. The term also encompasses polynucleotides that are distinguished from a reference polynucleotide by the addition, deletion or substitution of at least one nucleotide. Accordingly, the term "polynucleotide variant" includes polynucleotides in which one or more nucleotides have been added or deleted, or replaced with different nucleotides. In this regard, it is well understood in the art that certain alterations inclusive of mutations, additions, deletions and substitutions can be made to a reference polynucleotide whereby the altered polynucleotide retains the biological function or activity of the reference polynucleotide. The term "polynucleotide variant" also includes naturally occurring allelic variants. The terms "peptide variant" and "polypeptide variant" and the like refer to peptides and polypeptides that are distinguished from a reference peptide or polypeptide by the addition, deletion or substitution of at least one amino acid residue. In certain examples, a peptide or polypeptide variant is distinguished from a reference peptide or polypeptide by one or more substitutions, which may be conservative or non-conservative. In certain examples, the peptide or polypeptide variant comprises conservative substitutions and, in this regard, it is well understood in the art that some amino acids may be changed to others with broadly similar properties without changing the nature of the activity of the peptide or polypeptide. Peptide and polypeptide variants also encompass peptides and polypeptides in which one or more amino acids have been added or deleted, or replaced with different amino acid residues.

"Prevention" includes reduction of risk, incidence and/or severity of a condition or disorder. The terms "treatment" and "treat" include both prophylactic or preventive treatment (that prevent and/or slow the development of a targeted pathologic condition or disorder) and curative, therapeutic or disease-modifying treatment, including therapeutic measures that cure, slow down, lessen symptoms of, and/or halt progression of a pathologic condition or disorder; and treatment of patients at risk of contracting a disease or suspected to have contracted a disease, as well as patients who are ill or have been diagnosed as suffering from a disease or medical condition. The terms "treatment" and "treat" do not necessarily imply that a subject is treated until total recovery. The terms "treatment" and "treat" also refer to the maintenance and/or promotion of health in an individual not suffering from a disease but who may be susceptible to the development of an unhealthy condition. The terms "treatment" and "treat" are also intended to include the potentiation or otherwise enhancement of one or more primary prophylactic or therapeutic measures. As non-limiting examples, a treatment can be performed by a patient, a caregiver, a doctor, a nurse, or another healthcare professional.

The term "recombinant polynucleotide" as used herein refers to a polynucleotide formed *in vitro* by the manipulation of nucleic acid into a form not normally found in nature. For example, the recombinant polynucleotide may be in the form of an expression vector. Generally, such expression vectors include transcriptional and translational regulatory nucleic acid operably linked to the nucleotide sequence.

The term "recombinant polypeptide" as used herein refers to a polypeptide made using recombinant techniques, ie, through the expression of a recombinant polynucleotide.

A "therapeutically effective amount" is at least the minimum concentration or amount required to affect a measurable improvement of a particular disease or condition. A therapeutically effective amount herein may vary according to factors such as the disease state, age, sex and weight of the patient. A therapeutically effective amount is also one in which any toxic or detrimental effects are outweighed by the therapeutically beneficial effects.

### Nucleic acid sequences

Sequences relevant to the present disclosure are set forth in Table 1.

**Table 1**

| **SEQ ID NO.** | **Description** | **Sequence** |
|---|---|---|
| 1 | Human transmembrane Klotho cDNA open reading frame | ATGCCCGCCAGCGCCCCGCCGCGCCGCCCGCGGCCGCCGCCGCCGTCG CTGTCGCTGCTGCTGCTGCTGCTGGGCCTGGGCGGCCGCCGCCTGCGT GCGGAGCCGGGCGACGGCGCGCAGACCTGGGCCCGTTTCTCGCGGCCT CCTGCCCCCGAGGCCGCGGGCCTCTTCCAGGGCACCTTCCCCGACGGC TTCCTCTGGGCCGTGGGCAGCGCCGCCTACCAGACCGAGGGCGGCTGG CAGCAGCACGGCAAGGGTGCGTCCATCTGGGATACGTTCACCCACCAC CCCCTGGCACCCCCGGGAGACTCCCGGAACGCCAGTCTGCCGTTGGGC GCCCCGTCGCCGCTGCAGCCCGCCACCGGGGACGTAGCCAGCGACAGC TACAACAACGTCTTCCGCGACACGGAGGCGCTGCGCGAGCTCGGGGTC ACTCACTACCGCTTCTCCATCTCGTGGGCGCGAGTGCTCCCCAATGGC AGCGCGGGCGTCCCCAACCGCGAGGGGCTGCGCTACTACCGGCGCCTG CTGGAGCGGCTGCGGGAGCTGGGCGTGCAGCCCGTGGTCACCCTGTAC CACTGGGACCTGCCCCAGCGCCTGCAGGACGCCTACGGCGGCTGGGCC AACCGCGCCCTGGCCGACCACTTCAGGGATTACGCGGAGCTCTGCTTC CGCCACTTCGGCGGTCAGGTCAAGTACTGGATCACCATCGACAACCCC TACGTGGTGGCCTGGCACGGCTACGCCACCGGGCGCCTGGCCCCCGGC ATCCGGGGCAGCCCGCGGCTCGGGTACCTGGTGGCGCACAACCTCCTC CTGGCTCATGCCAAAGTCTGGCATCTCTACAATACTTCTTTCCGTCCC ACTCAGGGAGGTCAGGTGTCCATTGCCCTAAGCTCTCACTGGATCAAT CCTCGAAGAATGACCGACCACAGCATCAAAGAATGTCAAAAATCTCTG GACTTTGTACTAGGTTGGTTTGCCAAACCCGTATTTATTGATGGTGAC TATCCCGAGAGCATGAAGAATAACCTTTCATCTATTCTGCCTGATTTT ACTGAATCTGAGAAAAAGTTCATCAAAGGAACTGCTGACTTTTTTGCT CTTTGCTTTGGACCCACCTTGAGTTTTCAACTTTTGGACCCTCACATG AAGTTCCGCCAATTGGAATCTCCCAACCTGAGGCAACTGCTTTCCTGG ATTGACCTTGAATTTAACCATCCTCAAATATTTATTGTGGAAAATGGC TGGTTTGTCTCAGGGACCACCAAGAGAGATGATGCCAAATATATGTAT TACCTCAAAAAGTTCATCATGGAAACCTTAAAAGCCATCAAGCTGGAT GGGGTGGATGTCATCGGGTATACCGCATGGTCCCTCATGGATGGTTTC GAGTGGCACAGAGGTTACAGCATCAGGCGTGGACTCTTCTATGTTGAC TTTCTAAGCCAGGACAAGATGTTGTTGCCAAAGTCTTCAGCCTTGTTC TACCAAAAGCTGATACAGAAAAATGGCTTCCCTCCTTTACCTGAAAAT CAGCCCCTAGAAGGGACATTTCCCTGTGACTTTGCTTGGGGAGTTGTT GACAACTACATTCAAGTAGATACCACTCTGTCTCAGTTTACCGACCTG AATGTTTACCTGTGGGATGTCCACCACAGTAAAAGGCTTATTAAAGTG GATGGGGTTGTGACCAAGAAGAGGAAATCCTACTGTGTTGACTTTGCT GCCATCCAGCCCCAGATCGCTTTACTCCAGGAAATGCACGTTACACAT TTTCGCTTCTCCCTGGACTGGGCCCTGATTCTCCCTCTGGGTAACCAG TCCCAGGTGAACCACACCATCCTGCAGTACTATCGCTGCATGGCCAGC GAGCTTGTCCGTGTCAACATCACCCCAGTGGTGGCCCTGTGGCAGCCT ATGGCCCCGAACCAAGGACTGCCGCGCCTCCTGGCCAGGCAGGGCGCC TGGGAGAACCCCTACACTGCCCTGGCCTTTGCAGAGTATGCCCGACTG TGCTTTCAAGAGCTCGGCCATCACGTCAAGCTTTGGATAACGATGAAT GAGCCGTATACAAGGAATATGACATACAGTGCTGGCCACAACCTTCTG AAGGCCCATGCCCTGGCTTGGCATGTGTACAATGAAAAGTTTAGGCAT GCTCAGAATGGGAAAATATCCATAGCCTTGCAGGCTGATTGGATAGAA CCTGCCTGCCCTTTCTCCCAAAAGGACAAAGAGGTGGCTGAGAGAGTT TTGGAATTTGACATTGGCTGGCTGGCTGAGCCCATTTTCGGCTCTGGA GATTATCCATGGGTGATGAGGGACTGGCTGAACCAAAGAAACAATTTT CTTCTTCCTTATTTCACTGAAGATGAAAAAAAGCTAATCCAGGGTACC TTTGACTTTTTGGCTTTAAGCCATTATACCACCATCCTTGTAGACTCA |
| | • KL-VS substitution SNPs underlined | |
| | | GAAAAAGAAGATCCAATAAAATACAATGATTACCTAGAAGTGCAAGAA ATGACCGACATCACGTGGCTCAACTCCCCCAGTCAGGTGGCGGTAGTG CCCTGGGGGTTGCGCAAAGTGCTGAACTGGCTGAAGTTCAAGTACGGA GACCTCCCCATGTACATAATATCCAATGGAATCGATGACGGGCTGCAT GCTGAGGACGACCAGCTGAGGGTGTATTATATGCAGAATTACATAAAC GAAGCTCTCAAAGCCCACATACTGGATGGTATCAATCTTTGCGGATAC TTTGCTTATTCGTTTAACGACCGCACAGCTCCGAGGTTTGGCCTCTAT CGTTATGCTGCAGATCAGTTTGAGCCCAAGGCATCCATGAAACATTAC AGGAAAATTATTGACAGCAATGGTTTCCCGGGCCCAGAAACTCTGGAA AGATTTTGTCCAGAAGAATTCACCGTGTGTACTGAGTGCAGTTTTTTT CACACCCGAAAGTCTTTACTGGCTTTCATAGCTTTTCTATTTTTTGCT TCTATTATTTCTCTCTCCCTTATATTTACTACTCGAAGAAAGGCAGA AGAAGTTACAAATAG |
| 2 | Human KL1+KL2 cDNA open reading frame | ATGCCCGCCAGCGCCCCGCCGCGCCGCCCGCGGCCGCCGCCGCCGTCG CTGTCGCTGCTGCTGGTGCTGCTGGCCTGGGCGGCCGCCGCCTGCGT GCGGAGCCGGGCGACGGCGCGCAGACCTGGGCCCGTTTCTCGCGGCCT CCTGCCCCCGAGGCCGCGGCCTCTTCCAGGGCACCTTCCCCGACGGC TTCCTCTGGGCCGTGGGCAGCGCCGCCTACCAGACCGAGGGCGGCTGG CAGCAGCACGGCAAGGGTGCGTCCATCTGGGATACGTTCACCCACCAC CCCCTGGCACCCCCGGGAGACTCCGGAACGCCAGTCTGCCGTTGGGC GCCCCGTCGCCGCTGCAGCCCGCCACCGGGGACGTAGCCAGCGACAGC TACAACAACGTCTTCCGCGACACGGAGGCGCTGCGCGAGCTCGGGGTC ACTCACTACCGCTTCTCCATCTCGTGGGCGCGAGTGCTCCCCAATGGC AGCGCGGGCGTCCCCAACCGCGAGGGGCTGCGCTACTACCGGCGCCTG CTGGAGCGGCTGCGGGAGCTGGGCGTGCAGCCCGTGGTCACCCTGTAC CACTGGGACCTGCCCCAGCGCCTGCAGGACGCCTACGGCGGCTGGGCC AACCGCGCCCTGGCCGACCACTTCAGGGATTACGCGGAGCTCTGCTTC CGCCACTTCGGCGGTCAGGTCAAGTACTGGATCACCATCGACAACCCC TACGTGGTGGCCTGGCACGGCTACGCCACCGGGCGCCTGGCCCCCGGC ATCCGGGGCAGCCCGCGGCTCGGGTACCTGGTGGCGCACAACCTCCTC CTGGCTCATGCCAAAGTCTGGCATCTCTACAATACTTCTTTCCGTCCC ACTCAGGGAGGTCAGGTGTCCATTGCCCTAAGCTCTCACTGGATCAAT CCTCGAAGAATGACCGACCACAGCATCAAAGAATGTCAAAAATCTCTG GACTTTGTACTAGGTTGGTTTGCCAAACCCGTATTTATTGATGGTGAC TATCCCGAGAGCATGAAGAATAACCTTTCATCTATTCTGCCTGATTTT ACTGAATCTGAGAAAAAGTTCATCAAAGGAACTGCTGACTTTTTTGCT CTTTGCTTTGGACCCACCTTGAGTTTTCAACTTTTGGACCCTCACATG AAGTTCCGCCAATTGGAATCTCCCAACCTGAGGCAACTGCTTTCCTGG ATTGACCTTGAATTTAACCATCCTCAAATATTTATTGTGGAAAATGGC TGGTTTGTCTCAGGGACCACCAAGAGAGATGATGCCAAATATATGTAT TACCTCAAAAAGTTCATCATGGAAACCTTAAAAGCCATCAAGCTGGAT GGGGTGGATGTCATCGGGTATACCGCATGGTCCCTCATGGATGGTTTC GAGTGGCACAGAGGTTACAGCATCAGGCGTGGACTCTTCTATGTTGAC TTTCTAAGCCAGGACAAGATGTTGTTGCCAAAGTCTTCAGCCTTGTTC TACCAAAAGCTGATAGAGAAAAATGGCTTCCCTCCTTTACCTGAAAAT CAGCCCCTAGAAGGGACATTTCCCTGTGACTTTGCTTGGGGAGTTGTT GACAACTACATTCAAGTAGATACCACTCTGTCTCAGTTTACCGACCTG AATGTTTACCTGTGGGATGTCCACCACAGTAAAAGGCTTATTAAAGTG GATGGGGTTGTGACCAAGAAGAGGAAATCCTACTGTGTTGACTTTGCT GCCATCCAGCCCCAGATCGCTTTACTCCAGGAAATGCACGTTACACAT TTTCGCTTCTCCCTGGACTGGGCCCTGATTCTCCCTCTGGGTAACCAG TCCCAGGTGAACCACACCATCCTGCAGTACTATCGCTGCATGGCCAGC GAGCTTGTCCGTGTCAACATCACCCCAGTGGTGGCCCTGTGGCAGCCT ATGGCCCCGAACCAAGGACTGCCGCGCCTCCTGGCCAGGCAGGGCGCC TGGGAGAACCCCTACACTGCCCTGGCCTTTGCAGAGTATGCCCGACTG |
| | | TGCTTTCAAGAGCTCGGCCATCACGTCAAGCTTTGGATAACGATGAAT GAGCCGTATACAAGGAATATGACATACAGTGCTGGCCACAACCTTCTG AAGGCCCATGCCCTGGCTTGGCATGTGTACAATGAAAAGTTTAGGCAT GCTCAGAATGGGAAAATATCCATAGCCTTGCAGGCTGATTGGATAGAA CCTGCCTGCCCTTTCTCCCAAAAGGACAAAGAGGTGGCTGAGAGAGTT TTGGAATTTGACATTGGCTGGCTGGCTGAGCCCATTTTCGGCTCTGGA GATTATCCATGGGTGATGAGGGACTGGCTGAACCAAAGAAACAATTTT CTTCTTCCTTATTTCACTGAAGATGAAAAAAAGCTAATCCAGGGTACC TTTGACTTTTTGGCTTTAAGCCATTATACCACCATCCTTGTAGACTCA GAAAAAGAAGATCCAATAAAATACAATGATTACCTAGAAGTGCAAGAA ATGACCGACATCACGTGGCTCAACTCCCCCAGTCAGGTGGCGGTAGTG CCCTGGGGGTTGCGCAAAGTGCTGAACTGGCTGAAGTTCAAGTACGGA GACCTCCCCATGTACATAATATCCAATGGAATCGATGACGGGCTGCAT GCTGAGGACGACCAGCTGAGGGTGTATTATATGCAGAATTACATAAAC GAAGCTCTCAAAGCCCACATACTGGATGGTATCAATCTTTGCGGATAC TTTGCTTATTCGTTTAACGACCGCACAGCTCCGAGGTTTGGCCTCTAT CGTTATGCTGCAGATCAGTTTGAGCCCAAGGCATCCATGAAACATTAC AGGAAAATTATTGACAGCAATGGTTTCCCGGGCCCAGAAACTCTGGAA AGATTTTGTCCAGAAGAATTCACCGTGTGTACTGAGTGCAGTTTTTTT CACACCCGAAAGTAG |
| 3 | Human KL1 cDNA open reading frame | ATGCCCGCCAGCGCCCCGCCGCGCCGCCCGCGGCCGCCGCCGCCGTCG CTGTCGCTGCTGCTGGTGCTGCTGGCCTGGCGGCCGCCGCCTGCGT GCGGAGCCGGGCGACGGCGCGCAGACCTGGGCCCGTTTCTCGCGGCCT CCTGCCCCCGAGGCCGCGGGCCTCTTCCAGGGCACCTTCCCCGACGGC TTCCTCTGGGCCGTGGGCAGCGCCGCCTACCAGACCGAGGGCGGCTGG CAGCAGCACGGCAAGGGTGCGTCCATCTGGGATACGTTCACCCACCAC CCCCTGGCACCCCCGGGAGACTCCCGGAACGCCAGTCTGCCGTTGGGC GCCCCGTCGCCGCTGCAGCCCGCCACCGGGGACGTAGCCAGCGACAGC TACAACAACGTCTTCCGCGACACGGAGGCGCTGCGCGAGCTCGGGGTC ACTCACTACCGCTTCTCCATCTCGTGGGCGCGAGTGCTCCCCAATGGC AGCGCGGGCGTCCCCAACCGCGAGGGGCTGCGCTACTACCGGCGCCTG CTGGAGCGGCTGCGGGAGCTGGGCGTGCAGCCCGTGGTCACCCTGTAC CACTGGGACCTGCCCCAGCGCCTGCAGGACGCCTACGGCGGCTGGGCC AACCGCGCCCTGGCCGACCACTTCAGGGATTACGCGGAGCTCTGCTTC CGCCACTTCGGCGGTCAGGTCAAGTACTGGATCACCATCGACAACCCC TACGTGGTGGCCTGGCACGGCTACGCCACCGGGCGCCTGGCCCCCGGC ATCCGGGGCAGCCCGCGGCTCGGGTACCTGGTGGCGCACAACCTCCTC CTGGCTCATGCCAAAGTCTGGCATCTCTACAATACTTCTTTCCGTCCC ACTCAGGGAGGTCAGGTGTCCATTGCCCTAAGCTCTCACTGGATCAAT CCTCGAAGAATGACCGACCACAGCATCAAAGAATGTCAAAAATCTCTG GACTTTGTACTAGGTTGGTTTGCCAAACCCGTATTTATTGATGGTGAC TATCCCGAGAGCATGAAGAATAACCTTTCATCTATTCTGCCTGATTTT ACTGAATCTGAGAAAAAGTTCATCAAAGGAACTGCTGACTTTTTTGCT CTTTGCTTTGGACCCACCTTGAGTTTTCAACTTTTGGACCCTCACATG AAGTTCCGCCAATTGGAATCTCCCAACCTGAGGCAACTGCTTTCCTGG ATTGACCTTGAATTTAACCATCCTCAAATATTTATTGTGGAAAATGGC TGGTTTGTCTCAGGGACCACCAAGAGAGATGATGCCAAATATATGTAT TACCTCAAAAAGTTCATCATGGAAACCTTAAAAGCCATCAAGCTGGAT GGGGTGGATGTCATCGGGTATACCGCATGGTCCCTCATGGATGGTTTC GAGTGGCACAGAGGTTACAGCATCAGGCGTGGACTCTTCTATGTTGAC TTTCTAAGCCAGGACAAGATGTTGTTGCCAAAGTCTTCAGCCTTGTTC TACCAAAAGCTGATAGAGAAAAATGGCTTCCCTCCTTTACCTGAAAAT CAGCCCCTAGAAGGGACATTTCCCTGTGACTTTGCTTGGGGAGTTGTT GACAACTACATTCAAGTATAG |
| 4 | Human secreted Klotho (sKL) cDNA open reading frame | ATGCCCGCCAGCGCCCCGCCGCGCCGCCCGCGGCCGCCGCCGCCGTCG CTGTCGCTGCTGCTGGTGCTGCTGGCCTGGGCGGCCGCCGCCTGCGT GCGGAGCCGGGCGACGGCGCGCAGACCTGGGCCCGTTTCTCGCGGCCT CCTGCCCCCGAGGCCGCGGCCTCTTCCAGGGCACCTTCCCCGACGGC TTCCTCTGGGCCGTGGGCAGCGCCGCCTACCAGACCGAGGGCGGCTGG CAGCAGCACGGCAAGGGTGCGTCCATCTGGGATACGTTCACCCACCAC CCCCTGGCACCCCCGGGAGACTCCCGGAACGCCAGTCTGCCGTTGGGC GCCCCGTCGCCGCTGCAGCCCGCCACCGGGGACGTAGCCAGCGACAGC TACAACAACGTCTTCCGCGACACGGAGGCGCTGCGCGAGCTCGGGGTC ACTCACTACCGCTTCTCCATCTCGTGGGCGCGAGTGCTCCCCAATGGC AGCGCGGGCGTCCCCAACCGCGAGGGGCTGCGCTACTACCGGCGCCTG CTGGAGCGGCTGCGGGAGCTGGGCGTGCAGCCCGTGGTCACCCTGTAC CACTGGGACCTGCCCCAGCGCCTGCAGGACGCCTACGGCGGCTGGGCC AACCGCGCCCTGGCCGACCACTTCAGGGATTACGCGGAGCTCTGCTTC CGCCACTTCGGCGGTCAGGTCAAGTACTGGATCACCATCGACAACCCC TACGTGGTGGCCTGGCACGGCTACGCCACCGGGCGCCTGGCCCCCGGC ATCCGGGGCAGCCCGCGGCTCGGGTACCTGGTGGCGCACAACCTCCTC CTGGCTCATGCCAAAGTCTGGCATCTCTACAATACTTCTTTCCGTCCC ACTCAGGGAGGTCAGGTGTCCATTGCCCTAAGCTCTCACTGGATCAAT CCTCGAAGAATGACCGACCACAGCATCAAAGAATGTCAAAAATCTCTG GACTTTGTACTAGGTTGGTTTGCCAAACCCGTATTTATTGATGGTGAC TATCCCGAGAGCATGAAGAATAACCTTTCATCTATTCTGCCTGATTTT ACTGAATCTGAGAAAAAGTTCATCAAAGGAACTGCTGACTTTTTTGCT CTTTGCTTTGGACCCACCTTGAGTTTTCAACTTTTGGACCCTCACATG AAGTTCCGCCAATTGGAATCTCCCAACCTGAGGCAACTGCTTTCCTGG ATTGACCTTGAATTTAACCATCCTCAAATATTTATTGTGGAAAATGGC TGGTTTGTCTCAGGGACCACCAAGAGAGATGATGCCAAATATATGTAT TACCTCAAAAAGTTCATCATGGAAACCTTAAAAGCCATCAAGCTGGAT GGGGTGGATGTCATCGGGTATACCGCATGGTCCCTCATGGATGGTTTC GAGTGGCACAGAGGTTACAGCATCAGGCGTGGACTCTTCTATGTTGAC TTTCTAAGCCAGGACAAGATGTTGTTGCCAAAGTCTTCAGCCTTGTTC TACCAAAAGCTGATAGAGAAAAATGGCTTCCCTCCTTTACCTGAAAAT CAGCCCCTAGAAGGGACATTTCCCTGTGACTTTGCTTGGGGAGTTGTT GACAACTACATTCAAGTAAGTCAGCTGACAAAACCAATCAGCAGTCTC ACCAAGCCCTATCACTAG |
| | • sKL tail underlined | |
| | • Used in cancer study | |
| 5 | Mouse transmembrane Klotho cDNA open reading frame | ATGCTAGCCCGCGCCCCTCCTCGCCGCCCGCCGCGGCTGGTGCTGCTC CGTTTGCTGTTGCTGCATCTGCTGCTGCTCGCCCTGCGCGCCCGCTGC CTGAGCGCTGAGCCGGGTCAGGGCGCGCAGACCTGGGCTCGCTTCGCG CGCGCTCCTGCCCCAGAGGCCGCTGGCCTCCTCCACGACACCTTCCCC GACGGTTTCCTCTGGGCGGTAGGCAGCGCCGCCTATCAGACCGAGGGC GGCTGGCGACAGCACGGCAAAGGCGCGTCCATCTGGGACACTTTCACC CATCACTCTGGGGCGGCCCCGTCCGACTCCCCGATCGTCGTGGCGCCG TCGGGTGCCCCGTCGCCTCCCCTGTCCTCCACTGGAGATGTGGCCAGC GATAGTTACAACAACGTCTACCGCGACACAGAGGGGCTGCGCGAACTG GGGGTCACCCACTACCGCTTCTCCATATCGTGGGCGCGGGTGCTCCCC AATGGCACCGCGGGCACTCCCAACCGCGAGGGGCTGCGCTACTACCGG CGGCTGCTGGAGCGGCTGCGGGAGCTGGGCGTGCAGCCGGTGGTTACC CTGTACCATTGGGACCTGCCACAGCGCCTGCAGGACACCTATGGCGGA TGGGCCAATCGCGCCCTGGCCGACCATTTCAGGGATTATGCCGAGCTC TGCTTCCGCCACTTCGGTGGTCAGGTCAAGTACTGGATCACCATTGAC AACCCCTACGTGGTGGCCTGGCACGGGTATGCCACCGGGCGCCTGGCC CCGGGCGTGAGGGGCAGCTCCAGGCTCGGGTACCTGGTTGCCCACAAC CTACTTTTGGCTCATGCCAAAGTCTGGCATCTCTACAACACCTCTTTC CGCCCCACACAGGGAGGCCGGGTGTCTATCGCCTTAAGCTCCCATTGG ATCAATCCTCGAAGAATGACTGACTATAATATCAGAGAATGCCAGAAG |
| | | TCTCTTGACTTTGTGCTAGGCTGGTTTGCCAAACCCATATTTATTGAT GGCGACTACCCAGAGAGTATGAAGAACAACCTCTCGTCTCTTCTGCCT GATTTACTGAATCTGAGAAGAGGCTCATCAGAGGAACTGCTGACTTT TTTGCTCTCTCCTTCGGACCAACCTTGAGCTTTCAGCTATTGGACCCT AACATGAAGTTCCGCCAATTGGAGTCTCCCAACCTGAGGCAGCTTCTG TCTTGGATAGATCTGGAATATAACCACCCTCCAATATTTATTGTGGAA AATGGCTGGTTTGTCTCGGGAACCACCAAAAGGGATGATGCCAAATAT ATGTATTATCTCAAGAAGTTCATAATGGAAACCTTAAAAGCAATCAGA CTGGATGGGGTCGACGTCATTGGGTACACCGCGTGGTCGCTCATGGAC GGTTTCGAGTGGCATAGGGGCTACAGCATCCGGCGAGGACTCTTCTAC GTTGACTTTCTGAGTCAGGACAAGGAGCTGTTGCCAAAGTCTTCGGCC TTGTTCTACCAAAAGCTGATAGAGGACAATGGCTTTCCTCCTTTACCT GAAAACCAGCCCCTTGAAGGGACATTTCCCTGTGACTTTGCTTGGGGA GTTGTTGACAACTACGTTCAAGTGGACACTACTCTCTCTCAGTTTACT GACCCGAATGTCTATCTGTGGGATGTGCATCACAGTAAGAGGCTTATT AAAGTAGACGGGGTTGTAGCCAAGAAGAGAAAACCTTACTGTGTTGAT TTCTCTGCCATCCGGCCTCAGATAACCTTACTTCGAGAAATGCGGGTC ACCCACTTTCGCTTCTCCCTGGACTGGGCCCTGATCTTGCCTCTGGGT AACCAGACCCAAGTGAACCACACGGTTCTGCACTTCTACCGCTGCATG ATCAGCGAGCTGGTGCACGCCAACATCACTCCAGTGGTGGCCCTGTGG CAGCCAGCAGCCCCGCACCAAGGCCTGCCACATGCCCTTGCAAAACAT GGGGCCTGGGAGAACCCGCACACTGCTCTGGCGTTTGCAGACTACGCA AACCTGTGTTTTAAAGAGTTGGGTCACTGGGTCAATCTCTGGATCACC ATGAACGAGCCAAACACACGGAACATGACCTATCGTGCCGGGCACCAC CTCCTGAGAGCCCATGCCTTGGCTTGGCATCTGTACGATGACAAGTTT AGGGCGGCTCAGAAAGGCAAAATATCCATCGCCTTGCAGGCTGACTGG ATAGAACCGGCCTGCCCTTTCTCTCAAAATGACAAAGAAGTGGCCGAG AGAGTTTTGGAATTTGATATAGGCTGGCTGGCAGAGCCTATTTTTGGT TCCGGAGATTATCCACGTGTGATGAGGGACTGGCTGAACCAAAAAAAC AATTTTCTTTTGCCCTATTTCACCGAAGATGAAAAAAAGCTAGTCCGG GGTTCCTTTGACTTCCTGGCGGTGAGTCATTACACCACCATTCTGGTA GACTGGGAAAAGGAGGATCCGATGAAATACAACGATTACTTGGAGGTA CAGGAGATGACTGACATCACATGGCTCAACTCTCCCAGTCAGGTGGCA GTGGTGCCTTGGGGGCTGCGCAAAGTGCTCAACTGGCTAAGGTTCAAG TACGGAGACCTCCCGATGTATGTGACAGCCAATGGAATCGATGATGAC CCCCACGCCGAGCAAGACTCACTGAGGATCTATTATATTAAGAATTAT GTGAATGAGGCTCTGAAAGCCTACGTGTTGGACGACATCAACCTTTGT GGCTACTTTGCGTATTCACTTAGTGATCGCTCAGCTCCCAAGTCTGGC TTTTATCGATATGCTGCGAATCAGTTTGAGCCCAAACCATCTATGAAA CATTACAGGAAAATTATTGACAGCAATGGCTTCTGTCCAGAAGAATAC ACTGTGTGCACCGAATGTGGATTTTTTCAAACCCGGAAGTCTTTGCTG GTCTTCATCTCGTTTCTTGTTTTTACTTTTATTATTTCTCTTGCTCTC ATTTTTCACTACTCCAAGAAAGGCCAGAGAAGTTATAAGTAG |
| 6 | Mouse KL1+KL2 cDNA open reading frame | ATGCTAGCCCGCGCCCCTCCTCGCCGCCCGCCGCGGCTGGTGCTGCTC CGTTTGCTGTTGCTGCATCTGCTGCTGCTCGCCCTGCGCGCCCGCTGC CTGAGCGCTGAGCCGGGTCAGGGCGCGCAGACCTGGGCTCGCTTCGCG CGCGCTCCTGCCCCAGAGGCCGCTGGCCTCCTCCACGACACCTTCCCC GACGGTTTCCTCTGGGCGGTAGGCAGCGCCGCCTATCAGACCGAGGGC GGCTGGCGACAGCACGGCAAAGGCGCGTCCATCTGGGACACTTTCACC CATCACTCTGGGGCGGCCCCGTCCGACTCCCCGATCGTCGTGGCGCCG TCGGGTGCCCCGTCGCCTCCCCTGTCCTCCACTGGAGATGTGGCCAGC GATAGTTACAACAACGTCTACCGCGACACAGAGGGGCTGCGCGAACTG GGGGTCACCCACTACCGCTTCTCCATATCGTGGGCGCGGGTGCTCCCC AATGGCACCGCGGGCACTCCCAACCGCGAGGGGCTGCGCTACTACCGG CGGCTGCTGGAGCGGCTGCGGGAGCTGGGCGTGCAGCCGGTGGTTACC |
| | | CTGTACCATTGGACCTGCCACAGCGCCTGCAGGACACCTATGGCGGA TGGGCCAATCGCGCCCTGGCCGACCATTTCAGGGATTATGCCGAGCTC TGCTTCCGCCACTTCGGTGGTCAGGTCAAGTACTGGATCACCATTGAC AACCCCTACGTGGTGGCCTGGCACGGGTATGCCACCGGGCGCCTGGCC CCGGGCGTGAGGGGCAGCTCCAGGCTCGGGTACCTGGTTGCCCACAAC CTACTTTTGGCTCATGCCAAAGTCTGGCATCTCTACAACACCTCTTTC CGCCCCACACAGGGAGGCCGGGTGTCTATCGCCTTAAGCTCCCATTGG ATCAATCCTCGAAGAATGACTGACTATAATATCAGAGAATGCCAGAAG TCTCTTGACTTTGTGCTAGGCTGGTTTGCCAAACCCATATTTATTGAT GGCGACTACCCAGAGAGTATGAAGAACAACCTCTCGTCTCTTCTGCCT GATTTTACTGAATCTGAGAAGAGGCTCATCAGAGGAACTGCTGACTTT TTTGCTCTCTCCTTCGGACCAACCTTGAGCTTTCAGCTATTGGACCCT AACATGAAGTTCCGCCAATTGGAGTCTCCCAACCTGAGGCAGCTTCTG TCTTGGATAGATCTGGAATATAACCACCCTCCAATATTTATTGTGGAA AATGGCTGGTTTGTCTCGGGAACCACCAAAAGGGATGATGCCAAATAT ATGTATTATCTCAAGAAGTTCATAATGGAAACCTTAAAAGCAATCAGA CTGGATGGGGTCGACGTCATTGGGTACACCGCGTGGTCGCTCATGGAC GGTTTCGAGTGGCATAGGGGCTACAGCATCCGGCGAGGACTCTTCTAC GTTGACTTTCTGAGTCAGGACAAGGAGCTGTTGCCAAAGTCTTCGGCC TTGTTCTACCAAAAGCTGATAGAGGACAATGGCTTTCCTCCTTTACCT GAAAACCAGCCCCTTGAAGGGACATTTCCCTGTGACTTTGCTTGGGGA GTTGTTGACAACTACGTTCAAGTGGACACTACTCTCTCTCAGTTTACT GACCCGAATGTCTATCTGTGGGATGTGCATCACAGTAAGAGGCTTATT AAAGTAGACGGGGTTGTAGCCAAGAAGAGAAAACCTTACTGTGTTGAT TTCTCTGCCATCCGGCCTCAGATAACCTTACTTCGAGAAATGCGGGTC ACCCACTTTCGCTTCTCCCTGGACTGGGCCCTGATCTTGCCTCTGGGT AACCAGACCCAAGTGAACCACACGGTTCTGCACTTCTACCGCTGCATG ATCAGCGAGCTGGTGCACGCCAACATCACTCCAGTGGTGGCCCTGTGG CAGCCAGCAGCCCCGCACCAAGGCCTGCCACATGCCCTTGCAAAACAT GGGGCCTGGGAGAACCCGCACACTGCTCTGGCGTTTGCAGACTACGCA AACCTGTGTTTTAAAGAGTTGGGTCACTGGGTCAATCTCTGGATCACC ATGAACGAGCCAAACACACGGAACATGACCTATCGTGCCGGGCACCAC CTCCTGAGAGCCCATGCCTTGGCTTGGCATCTGTACGATGACAAGTTT AGGGCGGCTCAGAAAGGCAAAATATCCATCGCCTTGCAGGCTGACTGG ATAGAACCGGCCTGCCCTTTCTCTCAAAATGACAAAGAAGTGGCCGAG AGAGTTTTGGAATTTGATATAGGCTGGCTGGCAGAGCCTATTTTTGGT TCCGGAGATTATCCACGTGTGATGAGGGACTGGCTGAACCAAAAAAAC AATTTTCTTTTGCCCTATTTCACCGAAGATGAAAAAAAGCTAGTCCGG GGTTCCTTTGACTTCCTGGCGGTGAGTCATTACACCACCATTCTGGTA GACTGGGAAAAGGAGGATCCGATGAAATACAACGATTACTTGGAGGTA CAGGAGATGACTGACATCACATGGCTCAACTCTCCCAGTCAGGTGGCA GTGGTGCCTTGGGGGCTGCGCAAAGTGCTCAACTGGCTAAGGTTCAAG TACGGAGACCTCCCGATGTATGTGACAGCCAATGGAATCGATGATGAC CCCCACGCCGAGCAAGACTCACTGAGGATCTATTATATTAAGAATTAT GTGAATGAGGCTCTGAAAGCCTACGTGTTGGACGACATCAACCTTTGT GGCTACTTTGCGTATTCACTTAGTGATCGCTCAGCTCCCAAGTCTGGC TTTTATCGATATGCTGCGAATCAGTTTGAGCCCAAACCATCTATGAAA CATTACAGGAAAATTATTGACAGCAATGGCTTCCTGGGTTCTGGAACA CTGGGAAGGTTTTGTCCAGAAGAATACACTGTGTGCACCGAATGTGGA TTTTTTCAAACCCGGAAGTAG |
| 7 | Mouse KL1 cDNA open reading frame | ATGCTAGCCCGCGCCCCTCCTCGCCGCCCGCCGCGGCTGGTGCTGCTC CGTTTGCTGTTGCTGCATCTGCTGCTGCTCGCCCTGCGCGCCCGCTGC CTGAGCGCTGAGCCGGGTCAGGGCGCGCAGACCTGGGCTCGCTTCGCG CGCGCTCCTGCCCCAGAGGCCGCTGGCCTCCTCCACGACACCTTCCCC GACGGTTTCCTCTGGGCGGTAGGCAGCGCCGCCTATCAGACCGAGGGC |
| | | GGCTGGCGACAGCACGGCAAAGGCGTCCATCTGGGACACTTTCACC CATCACTCTGGGGCGGCCCCGTCCGACTCCCCGATCGTCGTGGCGCCG TCGGGTGCCCCGTCGCCTCCCCTGTCCTCCACTGGAGATGTGGCCAGC GATAGTTACAACAACGTCTACCGCGACACAGAGGGGCTGCGCGAACTG GGGGTCACCCACTACCGCTTCTCCATATCGTGGGCGCGGGTGCTCCCC AATGGCACCGCGGGCACTCCCAACCGCGAGGGGCTGCGCTACTACCGG CGGCTGCTGGAGCGGCTGCGGGAGCTGGGCGTGCAGCCGGTGGTTACC CTGTACCATTGGGACCTGCCACAGCGCCTGCAGGACACCTATGGCGGA TGGGCCAATCGCGCCCTGGCCGACCATTTCAGGGATTATGCCGAGCTC TGCTTCCGCCACTTCGGTGGTCAGGTCAAGTACTGGATCACCATTGAC AACCCCTACGTGGTGGCCTGGCACGGGTATGCCACCGGGCGCCTGGCC CCGGGCGTGAGGGGCAGCTCCAGGCTCGGGTACCTGGTTGCCCACAAC CTACTTTTGGCTCATGCCAAAGTCTGGCATCTCTACAACACCTCTTTC CGCCCCACACAGGGAGGCCGGGTGTCTATCGCCTTAAGCTCCCATTGG ATCAATCCTCGAAGAATGACTGACTATAATATCAGAGAATGCCAGAAG TCTCTTGACTTTGTGCTAGGCTGGTTTGCCAAACCCATATTTATTGAT GGCGACTACCCAGAGAGTATGAAGAACAACCTCTCGTCTCTTCTGCCT GATTTTACTGAATCTGAGAAGAGGCTCATCAGAGGAACTGCTGACTTT TTTGCTCTCTCCTTCGGACCAACCTTGAGCTTTCAGCTATTGGACCCT AACATGAAGTTCCGCCAATTGGAGTCTCCCAACCTGAGGCAGCTTCTG TCTTGGATAGATCTGGAATATAACCACCCTCCAATATTTATTGTGGAA AATGGCTGGTTTGTCTCGGGAACCACCAAAAGGGATGATGCCAAATAT ATGTATTATCTCAAGAAGTTCATAATGGAAACCTTAAAAGCAATCAGA CTGGATGGGGTCGACGTCATTGGGTACACCGCGTGGTCGCTCATGGAC GGTTTCGAGTGGCATAGGGGCTACAGCATCCGGCGAGGACTCTTCTAC GTTGACTTTCTGAGTCAGGACAAGGAGCTGTTGCCAAAGTCTTCGGCC TTGTTCTACCAAAAGCTGATAGAGGACAATGGCTTTCCTCCTTTACCT GAAAACCAGCCCCTTGAAGGACATTTCCCTGTGACTTTGCTTGGGGA GTTGTTGACAACTACGTTCAAGTGTAG |
| 8 | Modified, HA-tagged mouse transmembrane Klotho cDNA open reading frame | ATGCTAGCCCGCGCCCCTCCTCGCCGCCCGCCGCGGCTGGTGCTGCTC CGTTTGCTGTTGCTGCATCTGCTGCTGCTCGCCCTGCGCGCCCGCTGC CTGAGCGCTGAGTACCCATACGATGTTCCAGATTACGCGCCGGGTCAG GGCGCGCAGACCTGGGCTCGCTTCGCGCGCGCTCCTGCCCCAGAGGCC GCTGGCCTCCTCCACGACACCTTCCCCGACGGTTTCCTCTGGGCGGTA GGCAGCGCCGCCTATCAGACCGAGGGCGGCTGGCGACAGCACGGCAAA GGCGCGTCCATCTGGGACACTTTCACCCATCACTCTGGGGCGGCCCCG TCCGACTCCCCGATCGTCGTGGCGCCGTCGGGTGCCCCGTCGCCTCCC CTGTCCTCCACTGGAGATGTGGCCAGCGATAGTTACAACAACGTCTAC CGCGACACAGAGGGGCTGCGCGAACTGGGGGTCACCCACTACCGCTTC TCCATATCGTGGGCGCGGGTGCTCCCCAATGGCACCGCGGGCACTCCC AACCGCGAGGGGCTGCGCTACTACCGGCGGCTGCTGGAGCGGCTGCGG GAGCTGGGCGTGCAGCCGGTGGTTACCCTGTACCATTGGGACCTGCCA CAGCGCCTGCAGGACACCTATGGCGGATGGGCCAATCGCGCCCTGGCC GACCATTTCAGGGATTATGCCGAGCTCTGCTTCCGCCACTTCGGTGGT CAGGTCAAGTACTGGATCACCATTGACAACCCCTACGTGGTGGCCTGG CACGGGTATGCCACCGGGCGCCTGGCCCCGGGCGTGAGGGGCAGCTCC AGGCTCGGGTACCTGGTTGCCCACAACCTACTTTTGGCTCATGCCAAA GTCTGGCATCTCTACAACACCTCTTTCCGCCCCACACAGGGAGGCCGG GTGTCTATCGCCTTAAGCTCCCATTGGATCAATCCTCGAAGAATGACT GACTATAATATCAGAGAATGCCAGAAGTCTCTTGACTTTGTGCTAGGC TGGTTTGCCAAACCCATATTTATTGATGGCGACTACCCAGAGAGTATG AAGAACAACCTCTCGTCTCTTCTGCCTGATTTTACTGAATCTGAGAAG AGGCTCATCAGAGGAACTGCTGACTTTTTTGCTCTCTCCTTCGGACCA ACCTTGAGCTTTCAGCTATTGGACCCTAACATGAAGTTCCGCCAATTG GAGTCTCCCAACCTGAGGCAGCTTCTGTCTTGGATAGATCTGGAATAT |
| | • Human codon optimised | |
| | • HA tag underlined | |
| | | AACCACCCTCCAATATTTATTGTGGAAAATGGCTGGTTTGTCTCGGGA ACCACCAAAAGGATGATGCCAAATATATGTATTATCTCAAGAAGTTC ATAATGGAAACCTTAAAAGCAATCAGACTGGATGGGGTCGACGTCATT GGGTACACCGCGTGGTCGCTCATGGACGGTTTCGAGTGGCATAGGGGC TACAGCATCCGGCGAGGACTCTTCTACGTTGACTTTCTGAGTCAGGAC AAGGAGCTGTTGCCAAAGTCTTCGGCCTTGTTCTACCAAAAGCTGATA GAGGACAATGGCTTTCCTCCTTTACCTGAAAACCAGCCCCTTGAAGGG ACATTTCCCTGTGACTTTGCTTGGGGAGTTGTTGACAACTACGTTCAA GTGGACACTACTCTCTCTCAGTTTACTGACCCGAATGTCTATCTGTGG GATGTGCATCACAGTAAGAGGCTTATTAAAGTAGACGGGGTTGTAGCC AAGAAGAGAAAACCTTACTGTGTTGATTTCTCTGCCATCCGGCCTCAG ATAACCTTACTTCGAGAAATGCGGGTCACCCACTTTCGCTTCTCCCTG GACTGGGCCCTGATCTTGCCTCTGGGTAACCAGACCCAAGTGAACCAC ACGGTTCTGCACTTCTACCGCTGCATGATCAGCGAGCTGGTGCACGCC AACATCACTCCAGTGGTGGCCCTGTGGCAGCCAGCAGCCCCGCACCAA GGCCTGCCACATGCCCTTGCAAAACATGGGGCCTGGGAGAACCCGCAC ACTGCTCTGGCGTTTGCAGACTACGCAAACCTGTGTTTTAAAGAGTTG GGTCACTGGGTCAATCTCTGGATCACCATGAACGAGCCAAACACACGG AACATGACCTATCGTGCCGGGCACCACCTCCTGAGAGCCCATGCCTTG GCTTGGCATCTGTACGATGACAAGTTTAGGGCGGCTCAGAAAGGCAAA ATATCCATCGCCTTGCAGGCTGACTGGATAGAACCGGCCTGCCCTTTC TCTCAAAATGACAAAGAAGTGGCCGAGAGAGTTTTGGAATTTGATATA GGCTGGCTGGCAGAGCCTATTTTTGGTTCCGGAGATTATCCACGTGTG ATGAGGGACTGGCTGAACCAAAAAAACAATTTTCTTTTGCCCTATTTC ACCGAAGATGAAAAAAAGCTAGTCCGGGGTTCCTTTGACTTCCTGGCG GTGAGTCATTACACCACCATTCTGGTAGACTGGGAAAAGGAGGATCCG ATGAAATACAACGATTACTTGGAGGTACAGGAGATGACTGACATCACA TGGCTCAACTCTCCCAGTCAGGTGGCAGTGGTGCCTTGGGGGCTGCGC AAAGTGCTCAACTGGCTAAGGTTCAAGTACGGAGACCTCCCGATGTAT GTGACAGCCAATGGAATCGATGATGACCCCCACGCCGAGCAAGACTCA CTGAGGATCTATTATATTAAGAATTATGTGAATGAGGCTCTGAAAGCC TACGTGTTGGACGACATCAACCTTTGTGGCTACTTTGCGTATTCACTT AGTGATCGCTCAGCTCCCAAGTCTGGCTTTTATCGATATGCTGCGAAT CAGTTTGAGCCCAAACCATCTATGAAACATTACAGGAAAATTATTGAC AGCAATGGCTTCCTGGGTTCTGGAACACTGGGAAGGTTTTGTCCAGAA GAATACACTGTGTGCACCGAATGTGGATTTTTTCAAACCCGGAAGTCT TTGCTGGTCTTCATCTCGTTTCTTGTTTTTACTTTTATTATTTCTCTT GCTCTCATTTTTCACTACTCCAAGAAAGGCCAGAGAAGTTATAAGTGA |
| 9 | • Modified, HA-tagged mouse KL1+KL2 cDNA open reading frame | ATGCTAGCCCGCGCCCCTCCTCGCCGCCCGCCGCGGCTGGTGCTGCTC CGTTTGCTGTTGCTGCATCTGCTGCTGCTCGCCCTGCGCGCCCGCTGC CTGAGCGCTGAGTACCCATACGATGTTCCAGATTACGCGCCGGGTCAG GGCGCGCAGACCTGGGCTCGCTTCGCGCGCGCTCCTGCCCCAGAGGCC GCTGGCCTCCTCCACGACACCTTCCCCGACGGTTTCCTCTGGCGGTA GGCAGCGCCGCCTATCAGACCGAGGGCGGCTGGCGACAGCACGGCAAA GGCGCGTCCATCTGGGACACTTTCACCCATCACTCTGGGGCGGCCCCG TCCGACTCCCCGATCGTCGTGGCGCCGTCGGGTGCCCCGTCGCCTCCC CTGTCCTCCACTGGAGATGTGGCCAGCGATAGTTACAACAACGTCTAC CGCGACACAGAGGGGCTGCGCGAACTGGGGGTCACCCACTACCGCTTC TCCATATCGTGGGCGCGGGTGCTCCCCAATGGCACCGCGGGCACTCCC AACCGCGAGGGGCTGCGCTACTACCGGCGGCTGCTGGAGCGGCTGCGG GAGCTGGGCGTGCAGCCGGTGGTTACCCTGTACCATTGGGACCTGCCA CAGCGCCTGCAGGACACCTATGGCGGATGGGCCAATCGCGCCCTGGCC GACCATTTCAGGGATTATGCCGAGCTCTGCTTCCGCCACTTCGGTGGT CAGGTCAAGTACTGGATCACCATTGACAACCCCTACGTGGTGGCCTGG CACGGGTATGCCACCGGGCGCCTGGCCCCGGGCGTGAGGGGCAGCTCC |
| | • Human codon optimised | |
| | • HA tag underlined | |
| | | AGGCTCGGGTACCTGGTTGCCCACAACCTACTTTTGGCTCATGCCAAA GTCTGGCATCTCTACAACACCTCTTTCCGCCCACACAGGGAGGCCGG GTGTCTATCGCCTTAAGCTCCCATTGGATCAATCCTCGAAGAATGACT GACTATAATATCAGAGAATGCCAGAAGTCTCTTGACTTTGTGCTAGGC TGGTTTGCCAAACCCATATTTATTGATGGCGACTACCCAGAGAGTATG AAGAACAACCTCTCGTCTCTTCTGCCTGATTTTACTGAATCTGAGAAG AGGCTCATCAGAGGAACTGCTGACTTTTTTGCTCTCTCCTTCGGACCA ACCTTGAGCTTTCAGCTATTGGACCCTAACATGAAGTTCCGCCAATTG GAGTCTCCCAACCTGAGGCAGCTTCTGTCTTGGATAGATCTGGAATAT AACCACCCTCCAATATTTATTGTGGAAAATGGCTGGTTTGTCTCGGGA ACCACCAAAAGGGATGATGCCAAATATATGTATTATCTCAAGAAGTTC ATAATGGAAACCTTAAAAGCAATCAGACTGGATGGGGTCGACGTCATT GGGTACACCGCGTGGTCGCTCATGGACGGTTTCGAGTGGCATAGGGGC TACAGCATCCGGCGAGGACTCTTCTACGTTGACTTTCTGAGTCAGGAC AAGGAGCTGTTGCCAAAGTCTTCGGCCTTGTTCTACCAAAAGCTGATA GAGGACAATGGCTTTCCTCCTTTACCTGAAAACCAGCCCCTTGAAGGG ACATTTCCCTGTGACTTTGCTTGGGGAGTTGTTGACAACTACGTTCAA GTGGACACTACTCTCTCTCAGTTTACTGACCCGAATGTCTATCTGTGG GATGTGCATCACAGTAAGAGGCTTATTAAAGTAGACGGGGTTGTAGCC AAGAAGAGAAAACCTTACTGTGTTGATTTCTCTGCCATCCGGCCTCAG ATAACCTTACTTCGAGAAATGCGGGTCACCCACTTTCGCTTCTCCCTG GACTGGGCCCTGATCTTGCCTCTGGGTAACCAGACCCAAGTGAACCAC ACGGTTCTGCACTTCTACCGCTGCATGATCAGCGAGCTGGTGCACGCC AACATCACTCCAGTGGTGGCCCTGTGGCAGCCAGCAGCCCCGCACCAA GGCCTGCCACATGCCCTTGCAAAACATGGGGCCTGGGAGAACCCGCAC ACTGCTCTGGCGTTTGCAGACTACGCAAACCTGTGTTTTAAAGAGTTG GGTCACTGGGTCAATCTCTGGATCACCATGAACGAGCCAAACACACGG AACATGACCTATCGTGCCGGCACCACCTCCTGAGAGCCCATGCCTTG GCTTGGCATCTGTACGATGACAAGTTTAGGGCGGCTCAGAAAGGCAAA ATATCCATCGCCTTGCAGGCTGACTGGATAGAACCGGCCTGCCCTTTC TCTCAAAATGACAAAGAAGTGGCCGAGAGAGTTTTGGAATTTGATATA GGCTGGCTGGCAGAGCCTATTTTTGGTTCCGGAGATTATCCACGTGTG ATGAGGGACTGGCTGAACCAAAAAAACAATTTTCTTTTGCCCTATTTC ACCGAAGATGAAAAAAAGCTAGTCCGGGGTTCCTTTGACTTCCTGGCG GTGAGTCATTACACCACCATTCTGGTAGACTGGGAAAAGGAGGATCCG ATGAAATACAACGATTACTTGGAGGTACAGGAGATGACTGACATCACA TGGCTCAACTCTCCCAGTCAGGTGGCAGTGGTGCCTTGGGGGCTGCGC AAAGTGCTCAACTGGCTAAGGTTCAAGTACGGAGACCTCCCGATGTAT GTGACAGCCAATGGAATCGATGATGACCCCCACGCCGAGCAAGACTCA CTGAGGATCTATTATATTAAGAATTATGTGAATGAGGCTCTGAAAGCC TACGTGTTGGACGACATCAACCTTTGTGGCTACTTTGCGTATTCACTT AGTGATCGCTCAGCTCCCAAGTCTGGCTTTTATCGATATGCTGCGAAT CAGTTTGAGCCCAAACCATCTATGAAACATTACAGGAAAATTATTGAC AGCAATGGCTTCCTGGGTTCTGGAACACTGGGAAGGTTTTGTCCAGAA GAATACACTGTGTGCACCGAATGTGGATTTTTTCAAACCCGGAAGTGA |
| 10 | • Modified, HA-tagged mouse transmembrane Klotho mRNA | CACAUUUGCUUCUGACAUAGUUGUGUUGACUCACAACCCCAGAAACAG ACAUCAUGCUAGCCCGCGCCCCUCCUCGCCGCCCGCCGCGGCUGGUGC UGCUCCGUUUGCUGUUGCUGCAUCUGCUGCUGCUCGCCCUGCGCGCCC GCUGCCUGAGCGCUGAGUACCCAUACGAUGUUCCAGAUUACGCGCCGG GUCAGGGCGCGCAGACCUGGGCUCGCUUCGCGCGCGCUCCUGCCCCAG AGGCCGCUGGCCUCCUCCACGACACCUUCCCCGACGGUUUCCUCUGGG CGGUAGGCAGCGCCGCCUAUCAGACCGAGGGCGGCUGGCGACAGCACG GCAAAGGCGCGUCCAUCUGGGACACUUUCACCCAUCACUCUGGGGCGG CCCCGUCCGACUCCCCGAUCGUCGUGGCGCCGUCGGGUGCCCCGUCGC CUCCCCUGUCCUCCACUGGAGAUGUGGCCAGCGAUAGUUACAACAACG |
| | • Corresponds to SEQ ID NO. 8 | |
| | • Human codon optimised | |
| | • 5'UTR and 3'UTR underlined | UCUACCGCGACACAGAGGGCUGCGCGAACUGGGGUCACCCACUACC GCUUCUCCAUAUCGUGGGCGCGGGUGCUCCCCAAUGGCACCGCGGGCA CUCCCAACCGCGAGGGGCUGCGCUACUACCGGCGGCUGCUGGAGCGGC UGCGGGAGCUGGGCGUGCAGCCGGUGGUUACCCUGUACCAUUGGACC UGCCACAGCGCCUGCAGGACACCUAUGGCGGAUGGGCCAAUCGCGCCC UGGCCGACCAUUUCAGGGAUUAUGCCGAGCUCUGCUUCCGCCACUUCG GUGGUCAGGUCAAGUACUGGAUCACCAUUGACAACCCCUACGUGGUGG CCUGGCACGGGUAUGCCACCGGGCGCCUGGCCCCGGGCGUGAGGGGCA GCUCCAGGCUCGGGUACCUGGUUGCCCACAACCUACUUUUGGCUCAUG CCAAAGUCUGGCAUCUCUACAACACCUCUUUCCGCCCCACACAGGGAG GCCGGGUGUCUAUCGCCUUAAGCUCCCAUUGGAUCAAUCCUCGAAGAA UGACUGACUAUAAUAUCAGAGAAUGCCAGAAGUCUCUUGACUUUGUGC UAGGCUGGUUUGCCAAACCCAUAUUUAUUGAUGGCGACUACCCAGAGA GUAUGAAGAACAACCUCUCGUCUCUUCUGCCUGAUUUUACUGAAUCUG AGAAGAGGCUCAUCAGAGGAACUGCUGACUUUUUUGCUCUCUCCUUCG GACCAACCUUGAGCUUUCAGCUAUUGGACCCUAACAUGAAGUUCCGCC AAUUGGAGUCUCCCAACCUGAGGCAGCUUCUGUCUUGGAUAGAUCUGG AAUAUAACCACCCUCCAAUAUUUAUUGUGGAAAAUGGCUGGUUUGUCU CGGGAACCACCAAAAGGGAUGAUGCCAAAUAUAUGUAUUAUCUCAAGA AGUUCAUAAUGGAAACCUUAAAAGCAAUCAGACUGGAUGGGGUCGACG UCAUUGGGUACACCGCGUGGUCGCUCAUGGACGGUUUCGAGUGGCAUA GGGGCUACAGCAUCCGGCGAGGACUCUUCUACGUUGACUUUCUGAGUC AGGACAAGGAGCUGUUGCCAAAGUCUUCGGCCUUGUUCUACCAAAAGC UGAUAGAGGACAAUGGCUUUCCUCCUUUACCUGAAAACCAGCCCCUUG AAGGGACAUUUCCCUGUGACUUUGCUUGGGGAGUUGUUGACAACUACG UUCAAGUGGACACUACUCUCUCUCAGUUUACUGACCCGAAUGUCUAUC UGUGGGAUGUGCAUCACAGUAAGAGGCUUAUUAAAGUAGACGGGGUUG UAGCCAAGAAGAGAAAACCUUACUGUGUUGAUUUCUCUGCCAUCCGGC CUCAGAUAACCUUACUUCGAGAAAUGCGGGUCACCCACUUUCGCUUCU CCCUGGACUGGGCCCUGAUCUUGCCUCUGGGUAACCAGACCCAAGUGA ACCACACGGUUCUGCACUUCUACCGCUGCAUGAUCAGCGAGCUGGUGC ACGCCAACAUCACUCCAGUGGUGGCCCUGUGGCAGCCAGCAGCCCCGC ACCAAGGCCUGCCACAUGCCCUUGCAAAACAUGGGGCCUGGGAGAACC CGCACACUGCUCUGGCGUUUGCAGACUACGCAAACCUGUGUUUAAAG AGUUGGGUCACUGGGUCAAUCUCUGGAUCACCAUGAACGAGCCAAACA CACGGAACAUGACCUAUCGUGCCGGGCACCACCUCCUGAGAGCCCAUG CCUUGGCUUGGCAUCUGUACGAUGACAAGUUUAGGGCGGCUCAGAAAG GCAAAAUAUCCAUCGCCUUGCAGGCUGACUGGAUAGAACCGGCCUGCC CUUUCUCUCAAAAUGACAAAGAAGUGGCCGAGAGAGUUUUGGAAUUUG AUAUAGGCUGGCUGGCAGAGCCUAUUUUUGGUUCCGGAGAUUAUCCAC GUGUGAUGAGGGACUGGCUGAACCAAAAAAACAAUUUUCUUUUGCCCU AUUUCACCGAAGAUGAAAAAAAGCUAGUCCGGGGUUCCUUUGACUUCC UGGCGGUGAGUCAUUACACCACCAUUCUGGUAGACUGGGAAAAGGAGG AUCCGAUGAAAUACAACGAUUACUUGGAGGUACAGGAGAUGACUGACA UCACAUGGCUCAACUCUCCCAGUCAGGUGGCAGUGGUGCCUUGGGGGC UGCGCAAAGUGCUCAACUGGCUAAGGUUCAAGUACGGAGACCUCCCGA UGUAUGUGACAGCCAAUGGAAUCGAUGAUGACCCCCACGCCGAGCAAG ACUCACUGAGGAUCUAUUAUAUUAAGAAUUAUGUGAAUGAGGCUCUGA AAGCCUACGUGUUGGACGACAUCAACCUUUGUGGCUACUUUGCGUAUU CACUUAGUGAUCGCUCAGCUCCCAAGUCUGGCUUUUAUCGAUAUGCUG CGAAUCAGUUUGAGCCCAAACCAUCUAUGAAACAUUACAGGAAAAUUA UUGACAGCAAUGGCUUCCUGGGUUCUGGAACACUGGGAAGGUUUUGUC CAGAAGAAUACACUGUGUGCACCGAAUGUGGAUUUUUUCAAACCCGGA AGUCUUUGCUGGUCUUCAUCUCGUUUCUUGUUUUUACUUUUAUUAUUU CUCUUGCUCUCAUUUUUCACUACUCCAAGAAAGGCCAGAGAAGUUAUA |
| | | AGUGAACCCCCUUUCCUGCUCUUGCCUGUGAACAAUGGUUAAUUGUUC CCAAGAGAGCAUCUGUCAGUUGUUGGCAAAAUGAUAAAGACAUUUGAA AAUCUGUCUUCUGACAAAUAAAAAGCAUUUAUUUCACUGCAAUGAUGU UUUAAAAAAAAAAAAAAAAAAAAAAAAAAAAAAAAAAA |
| 11 | • Modified, HA-tagged mouse KL1+KL2 mRNA | CACAUUUGCUUCUGACAUAGUUGUGUUGACUCACAACCCCAGAAACAG ACAUCAUGCUAGCCCGCGCCCUCCUCGCCGCCCGCCGCGGCUGGUGC UGCUCCGUUUGCUGUUGCUGCAUCUGCUGCUGCUCGCCCUGCGCGCCC GCUGCCUGAGCGCUGAGUACCCAUACGAUGUUCCAGAUUACGCGCCGG GUCAGGGCGCGCAGACCUGGGCUCGCUUCGCGCGCGCUCCUGCCCCAG AGGCCGCUGGCCUCCUCCACGACACCUUCCCCGACGGUUUCCUCUGGG CGGUAGGCAGCGCCGCCUAUCAGACCGAGGGCGGCUGGCGACAGCACG GCAAAGGCGCGUCCAUCUGGGACACUUUCACCCAUCACUCUGGGGCGG CCCCGUCCGACUCCCCGAUCGUCGUGGCGCCGUCGGGUGCCCCGUCGC CUCCCCCUGUCCUCCACUGGAGAUGUGGCCAGCGAUAGUUACAACAACG UCUACCGCGACACAGAGGGGCUGCGCGAACUGGGGGUCACCCACUACC GCUUCUCCAUAUCGUGGGCGCGGGUGCUCCCCAAUGGCACCGCGGGCA CUCCCAACCGCGAGGGGCUGCGCUACUACCGGCGGCUGCUGGAGCGGC UGCGGGAGCUGGGCGUGCAGCCGGUGGUUACCCUGUACCAUUGGGACC UGCCACAGCGCCUGCAGGACACCUAUGGCGGAUGGGCCAAUCGCGCCC UGGCCGACCAUUUCAGGGAUUAUGCCGAGCUCUGCUUCCGCCACUUCG GUGGUCAGGUCAAGUACUGGAUCACCAUUGACAACCCCUACGUGGUGG CCUGGCACGGGUAUGCCACCGGGCGCCUGGCCCCGGGCGUGAGGGGCA GCUCCAGGCUCGGGUACCUGGUUGCCCACAACCUACUUUUGGCUCAUG CCAAAGUCUGGCAUCUCUACAACACCUCUUUCCGCCCCACACAGGGAG GCCGGGUGUCUAUCGCCUUAAGCUCCCAUUGGAUCAAUCCUCGAAGAA UGACUGACUAUAAUAUCAGAGAAUGCCAGAAGUCUCUUGACUUUGUGC UAGGCUGGUUUGCCAAACCCAUAUUUAUUGAUGGCGACUACCCAGAGA GUAUGAAGAACAACCUCUCGUCUCUUCUGCCUGAUUUUACUGAAUCUG AGAAGAGGCUCAUCAGAGGAACUGCUGACUUUUUUGCUCUCUCCUUCG GACCAACCUUGAGCUUUCAGCUAUUGGACCCUAACAUGAAGUUCCGCC AAUUGGAGUCUCCCAACCUGAGGCAGCUUCUGUCUUGGAUAGAUCUGG AAUAUAACCACCCUCCAAUAUUUAUUGUGGAAAAUGGCUGGUUUGUCU CGGGAACCACCAAAAGGGAUGAUGCCAAAUAUAUGUAUUAUCUCAAGA AGUUCAUAAUGGAAACCUUAAAAGCAAUCAGACUGGAUCGGCUCCACC UCAUUGGGUACACCGCGUGGUCGCUCAUGGACGGUUUCGAGUGGCAUA GGGGCUACAGCAUCCGGCGAGGACUCUUCUACGUUGACUUUCUGAGUC AGGACAAGGAGCUGUUGCCAAAGUCUUCGGCCUUGUUCUACCAAAAGC UGAUAGAGGACAAUGGCUUUCCUCCUUUACCUGAAAACCAGCCCCUUG AAGGGACAUUUCCCUGUGACUUUGCUUGGGGAGUUGUUGACAACUACG UUCAAGUGGACACUACUCUCUCUCAGUUUACUGACCCGAAUGUCUAUC UGUGGGAUGUGCAUCACAGUAAGAGGCUUAUUAAAGUAGACGGGGUUG UAGCCAAGAAGAGAAAACCUUACUGUGUUGAUUUCUCUGCCAUCCGGC CUCAGAUAACCUUACUUCGAGAAAUGCGGGUCACCCACUUUCGCUUCU CCCUGGACUGGGCCCUGAUCUUGCCUCUGGGUAACCAGACCCAAGUGA ACCACACGGUUCUGCACUUCUACCGCUGCAUGAUCAGCGAGCUGGUGC ACGCCAACAUCACUCCAGUGGUGGCCCUGUGGCAGCCAGCAGCCCCGC ACCAAGGCCUGCCACAUGCCCUUGCAAAACAUGGGGCCUGGGAGAACC CGCACACUGCUCUGGCGUUUGCAGACUACGCAAACCUGUGUUUUAAAG AGUUGGGUCACUGGGUCAAUCUCUGGAUCACCAUGAACGAGCCAAACA CACGGAACAUGACCUAUCGUGCCGGGCACCACCUCCUGAGAGCCCAUG CCUUCGCUUCGCCAUCUCUACCAUGACAACUUUAGCCCCCCUCACAAAC GCAAAAUAUCCAUCGCCUUGCAGGCUGACUGGAUAGAACCGGCCUGCC CUUUCUCUCAAAAUGACAAAGAAGUGGCCGAGAGAGUUUUGGAAUUUG AUAUAGGCUGGCUGGCAGAGCCUAUUUUUGGUUCCGGAGAUUAUCCAC GUGUGAUGAGGGACUGGCUGAACCAAAAAAACAAUUUUCUUUUGCCCU |
| | • Human codon optimised | |
| | • Corresponds to SEQ ID NO. 9 | |
| | • 5' UTR and 3'UTR underlined | |
| | | AUUUCACCGAAGAUGAAAAAAGCUAGUCCGGGUUCCUUUGACUUCC UGGCGGUGAGUCAUUACACCACCAUUCUGGUAGACUGGGAAAAGGAGG AUCCGAUGAAAUACAACGAUUACUUGGAGGUACAGGAGAUGACUGACA UCACAUGGCUCAACUCUCCCAGUCAGGUGGCAGUGGUGCCUUGGGGGC UGCGCAAAGUGCUCAACUGGCUAAGGUUCAAGUACGGAGACCUCCCGA UGUAUGUGACAGCCAAUGGAAUCGAUGAUGACCCCCACGCCGAGCAAG ACUCACUGAGGAUCUAUUAUAUUAAGAAUUAUGUGAAUGAGGCUCUGA AAGCCUACGUGUUGGACGACAUCAACCUUUGUGGCUACUUUGCGUAUU CACUUAGUGAUCGCUCAGCUCCCAAGUCUGGCUUUUAUCGAUAUGCUG CGAAUCAGUUUGAGCCCAAACCAUCUAUGAAACAUUACAGGAAAAUUA UUGACAGCAAUGGCUUCCUGGGUUCUGGAACACUGGGAAGGUUUUGUC CAGAAGAAUACACUGUGUGCACCGAAUGUGGAUUUUUUCAAACCCGGA AGUGAACCCCCUUUCCUGCUCUUGCCUGUGAACAAUGGUUAAUUGUUC CCAAGAGAGCAUCUGUCAGUUGUUGGCAAAAUGAUAAAGACAUUUGAA AAUCUGUCUUCUGACAAUAAAAGCAUUUAUUUCACUGCAAUGAUGU UUUAAAAAAAAAAAAAAAAAAAAAAAAAAAAAAAA |
| 12 | Human secreted Klotho | MPASAPPRRPRPPPPSLSLLLVLLGLGGRRLRAEPGDGAQTWARFSRP PAPEAAGLFQGTFPDGFLWAVGSAAYQTEGGWQQHGKGASIWDTFTHH PLAPPGDSRNASLPLGAPSPLQPATGDVASDSYNNVFRDTEALRELGV THYRFSISWARVLPNGSAGVPNREGLRYYRRLLERLRELGVQPVVTLY HWDLPQRLQDAYGGWANRALADHFRDYAELCFRHFGGQVKYWITIDNP YVVAWHGYATGRLAPGIRGSPRLGYLVAHNLLLAHAKVWHLYNTSFRP TQGGQVSIALSSHWINPRRMTDHSIKECQKSLDFVLGWFAKPVFIDGD YPESMKNNLSSILPDFTESEKKFIKGTADFFALCFGPTLSFQLLDPHM KFRQLESPNLRQLLSWIDLEFNHPQIFIVENGWFVSGTTKRDDAKYMY YLKKFIMETLKAIKLDGVDVIGYTAWSLMDGFEWHRGYSIRRGLFYVD FLSQDKMLLPKSSALFYQKLIEKNGFPPLPENQPLEGTFPCDFAWGVV DNYIQVSQLTKPISSLTKPYH |
| | • Translation of SEQ ID NO. 4 | |
| 13 | Modified, HA-tagged mouse transmembrane Klotho | MLARAPPRRPPRLVLLRLLLLHLLLLALRARCLSAEYPYDVPDYAPGQ GAQTWARFARAPAPEAAGLLHDTFPDGFLWAVGSAAYQTEGGWRQHGK GASIWDTFTHHSGAAPSDSPIVVAPSGAPSPPLSSTGDVASDSYNNVY RDTEGLRELGVTHYRFSISWARVLPNGTAGTPNREGLRYYRRLLERLR ELGVQPVVTLYHWDLPQRLQDTYGGWANRALADHFRDYAELCFRHFGG QVKYWITIDNPYVVAWHGYATGRLAPGVRGSSRLGYLVAHNLLLAHAK VWHLYNTSFRPTQGGRVSIALSSHWINPRRMTDYNIRECQKSLDFVLG WFAKPIFIDGDYPESMKNNLSSLLPDFTESEKRLIRGTADFFALSFGP TLSFQLLDPNMKFRQLESPNLRQLLSWIDLEYNHPPIFIVENGWFVSG TTKRDDAKYMYYLKKFIMETLKAIRLDGVDVIGYTAWSLMDGFEWHRG YSIRRGLFYVDFLSQDKELLPKSSALFYQKLIEDNGFPPLPENQPLEG TFPCDFAWGVVDNYVQVDTTLSQFTDPNVYLWDVHHSKRLIKVDGVVA KKRKPYCVDFSAIRPQITLLREMRVTHFRFSLDWALILPLGNQTQVNH TVLHFYRCMISELVHANITPVVALWQPAAPHQGLPHALAKHGAWENPH TALAFADYANLCFKELGHWVNLWITMNEPNTRNMTYRAGHHLLRAHAL AWHLYDDKFRAAQKGKISIALQADWIEPACPFSQNDKEVAERVLEFDI GWLAEPIFGSGDYPRVMRDWLNQKNNFLLPYFTEDEKKLVRGSFDFLA VSHYTTILVDWEKEDPMKYNDYLEVQEMTDITWLNSPSQVAVVPWGLR KVLNWLRFKYGDLPMYVTANGIDDDPHAEQDSLRIYYIKNYVNEALKA YVLDDINLCGYFAYSLSDRSAPKSGFYRYAANQFEPKPSMKHYRKIID SNGFLGSGTLGRFCPEEYTVCTECGFFQTRKSLLVFISFLVFTFIISL ALIFHYSKKGQRSYK |
| | • Translation of SEQ ID NO. 8 | |
| 14 | Modified, HA-tagged mouse KL1+KL2 | MLARAPPRRPPRLVLLRLLLLHLLLLALRARCLSAEYPYDVPDYAPGQ GAQTWARFARAPAPEAAGLLHDTFPDGFLWAVGSAAYQTEGGWRQHGK GASIWDTFTHHSGAAPSDSPIVVAPSGAPSPPLSSTGDVASDSYNNVY RDTEGLRELGVTHYRFSISWARVLPNGTAGTPNREGLRYYRRLLERLR |
| | • Translation of SEQ ID NO. 9 | ELGVQPVVTLYHWDLPQRLQDTYGGWANRALADHFRDYAELCFRHFGG QVKYWITIDNPYVVAWHGYATGRLAPGVRGSSRLGYLVAHNLLLAHAK VWHLYNTSFRPTQGGRVSIALSSHWINPRRMTDYNIRECQKSLDFVLG WFAKPIFIDGDYPESMKNNLSSLLPDFTESEKRLIRGTADFFALSFGP TLSFQLLDPNMKFRQLESPNLRQLLSWIDLEYNHPPIFIVENGWFVSG TTKRDDAKYMYYLKKFIMETLKAIRLDGVDVIGYTAWSLMDGFEWHRG YSIRRGLFYVDFLSQDKELLPKSSALFYQKLIEDNGFPPLPENQPLEG TFPCDFAWGVVDNYVQVDTTLSQFTDPNVYLWDVHHSKRLIKVDGVVA KKRKPYCVDFSAIRPQITLLREMRVTHFRFSLDWALILPLGNQTQVNH TVLHFYRCMISELVHANITPVVALWQPAAPHQGLPHALAKHGAWENPH TALAFADYANLCFKELGHWVNLWITMNEPNTRNMTYRAGHHLLRAHAL AWHLYDDKFRAAQKGKISIALQADWIEPACPFSQNDKEVAERVLEFDI GWLAEPIFGSGDYPRVMRDWLNQKNNFLLPYFTEDEKKLVRGSFDFLA VSHYTTILVDWEKEDPMKYNDYLEVQEMTDITWLNSPSQVAVVPWGLR KVLNWLRFKYGDLPMYVTANGIDDDPHAEQDSLRIYYIKNYVNEALKA YVLDDINLCGYFAYSLSDRSAPKSGFYRYAANQFEPKPSMKHYRKIID SNGFLGSGTLGRFCPEEYTVCTECGFFQTRK |
| 15 | Human transmembrane Klotho | |
| | • Signal sequence underlined | |
| | • Transmembrane domain lower case | |
| | • Cleavage sites in bold | |
| 16 | Human transmembrane Klotho cDNA open reading frame | ATGCCCGCCAGCGCCCCGCCGCGCCGCCCGCGGCCGCCGCCGCCGTCG CTGTCGCTGCTGCTGGTGCTGCTGGGCCTGGGCGGCCGCCGCCTGCGT GCGGAGCCGGGCGACGGCGCGCAGACCTGGGCCCGTTTCTCGCGGCCT CCTGCCCCCGAGGCCGCGGGCCTCTTCCAGGGCACCTTCCCCGACGGC TTCCTCTGGGCCGTGGGCAGCGCCGCCTACCAGACCGAGGGCGGCTGG CAGCAGCACGGCAAGGGTGCGTCCATCTGGGATACGTTCACCCACCAC CCCCTGGCACCCCCGGGAGACTCCCGGAACGCCAGTCTGCCGTTGGGC GCCCCGTCGCCGCTGCAGCCCGCCACCGGGGACGTAGCCAGCGACAGC TACAACAACGTCTTCCGCGACACGGAGGCGCTGCGCGAGCTCGGGGTC ACTCACTACCGCTTCTCCATCTCGTGGGCGCGAGTGCTCCCCAATGGC AGCGCGGGCGTCCCCAACCGCGAGGGGCTGCGCTACTACCGGCGCCTG CTGGAGCGGCTGCGGGAGCTGGGCGTGCAGCCCGTGGTCACCCTGTAC CACTGGGACCTGCCCCAGCGCCTGCAGGACGCCTACGGCGGCTGGGCC AACCGCGCCCTGGCCGACCACTTCAGGGATTACGCGGAGCTCTGCTTC CGCCACTTCGGCGGTCAGGTCAAGTACTGGATCACCATCGACAACCCC |
| | • KL-VS substitution SNPs underlined | |
| | | TACGTGGTGGCCTGGCACGGCTACGCCACCGGGCGCCTGGCCCCCGGC ATCCGGGGCAGCCCGCGGCTCGGGTACCTGGTGGCGCACAACCTCCTC CTGGCTCATGCCAAAGTCTGGCATCTCTACAATACTTCTTTCCGTCCC ACTCAGGGAGGTCAGGTGTCCATTGCCCTAAGCTCTCACTGGATCAAT CCTCGAAGAATGACCGACCACAGCATCAAAGAATGTCAAAAATCTCTG GACTTTGTACTAGGTTGGTTTGCCAAACCCGTATTTATTGATGGTGAC TATCCCGAGAGCATGAAGAATAACCTTTCATCTATTCTGCCTGATGTT ACTGAATCTGAGAAAAAGTTCATCAAAGGAACTGCTGACTTTTTTGCT CTTAGCTTTGGACCCACCTTGAGTTTTCAACTTTTGGACCCTCACATG AAGTTCCGCCAATTGGAATCTCCCAACCTGAGGCAACTGCTTTCCTGG ATTGACCTTGAATTTAACCATCCTCAAATATTTATTGTGGAAAATGGC TGGTTTGTCTCAGGGACCACCAAGAGAGATGATGCCAAATATATGTAT TACCTCAAAAAGTTCATCATGGAAACCTTAAAAGCCATCAAGCTGGAT GGGGTGGATGTCATCGGGTATACCGCATGGTCCCTCATGGATGGTTTC GAGTGGCACAGAGGTTACAGCATCAGGCGTGGACTCTTCTATGTTGAC TTTCTAAGCCAGGACAAGATGTTGTTGCCAAAGTCTTCAGCCTTGTTC TACCAAAAGCTGATAGAGAAAAATGGCTTCCCTCCTTTACCTGAAAAT CAGCCCCTAGAAGGGACATTTCCCTGTGACTTTGCTTGGGGAGTTGTT GACAACTACATTCAAGTAGATACCACTCTGTCTCAGTTTACCGACCTG AATGTTTACCTGTGGGATGTCCACCACAGTAAAAGGCTTATTAAAGTG GATGGGGTTGTGACCAAGAAGAGGAAATCCTACTGTGTTGACTTTGCT GCCATCCAGCCCCAGATCGCTTTACTCCAGGAAATGCACGTTACACAT TTTCGCTTCTCCCTGGACTGGGCCCTGATTCTCCCTCTGGGTAACCAG TCCCAGGTGAACCACACCATCCTGCAGTACTATCGCTGCATGGCCAGC GAGCTTGTCCGTGTCAACATCACCCCAGTGGTGGCCCTGTGGCAGCCT ATGGCCCCGAACCAAGGACTGCCGCGCCTCCTGGCCAGGCAGGGCGCC TGGGAGAACCCCTACACTGCCCTGGCCTTTGCAGAGTATGCCCGACTG TGCTTTCAAGAGCTCGGCCATCACGTCAAGCTTTGGATAACGATGAAT GAGCCGTATACAAGGAATATGACATACAGTGCTGGCCACAACCTTCTG AAGGCCCATGCCCTGGCTTGGCATGTGTACAATGAAAAGTTTAGGCAT GCTCAGAATGGGAAAATATCCATAGCCTTGCAGGCTGATTGGATAGAA CCTGCCTGCCCTTTCTCCCAAAAGGACAAAGAGGTGGCTGAGAGAGTT TTGGAATTTGACATTGGCTGGCTGGCTGAGCCCATTTTCGGCTCTGGA GATTATCCATGGGTGATGAGGGACTGGCTGAACCAAAGAAACAATTTT CTTCTTCCTTATTTCACTGAAGATGAAAAAAAGCTAATCCAGGGTACC TTTGACTTTTTGGCTTTAAGCCATTATACCACCATCCTTGTAGACTCA GAAAAAGAAGATCCAATAAAATACAATGATTACCTAGAAGTGCAAGAA ATGACCGACATCACGTGGCTCAACTCCCCCAGTCAGGTGGCGGTAGTG CCCTGGGGGTTGCGCAAAGTGCTGAACTGGCTGAAGTTCAAGTACGGA GACCTCCCCATGTACATAATATCCAATGGAATCGATGACGGGCTGCAT GCTGAGGACGACCAGCTGAGGGTGTATTATATGCAGAATTACATAAAC GAAGCTCTCAAAGCCCACATACTGGATGGTATCAATCTTTGCGGATAC TTTGCTTATTCGTTTAACGACCGCACAGCTCCGAGGTTTGGCCTCTAT CGTTATGCTGCAGATCAGTTTGAGCCCAAGGCATCCATGAAACATTAC AGGAAAATTATTGACAGCAATGGTTTCCCGGGCCCAGAAACTCTGGAA AGATTTTGTCCAGAAGAATTCACCGTGTGTACTGAGTGCAGTTTTTTT CACACCCGAAAGTCTTTACTGGCTTTCATAGCTTTTCTATTTTTTGCT TCTATTATTTCTCTCTCCCTTATATTTTACTACTCGAAGAAAGGCAGA AGAAGTTACAAATAG |
| 17 | Human transmembrane Klotho VS protein | MPASAPPRRPRPPPPSLSLLLVLLGLGGRRLRAEPGDGAQTWARFSRP PAPEAAGLFQGTFPDGFLWAVGSAAYQTEGGWQQHGKGASIWDTFTHH PLAPPGDSRNASLPLGAPSPLQPATGDVASDSYNNVFRDTEALRELGV THYRFSISWARVLPNGSAGVPNREGLRYYRRLLERLRELGVQPVVTLY HWDLPQRLQDAYGGWANRALADHFRDYAELCFRHFGGQVKYWITIDNP YVVAWHGYATGRLAPGIRGSPRLGYLVAHNLLLAHAKVWHLYNTSFRP |
| | • KL-VS substitutions underlined | TQGGQVSIALSSHWINPRRMTDHSIKECQKSLDFVLGWFAKPVFIDGD YPESMKNNLSSILPDVTESEKKFIKGTADFFALSFGPTLSFQLLDPHM KFRQLESPNLRQLLSWIDLEFNHPQIFIVENGWFVSGTTKRDDAKYMY YLKKFIMETLKAIKLDGVDVIGYTAWSLMDGFEWHRGYSIRRGLFYVD FLSQDKMLLPKSSALFYQKLIEKNGFPPLPENQPLEGTFPCDFAWGVV DNYIQVDTTLSQFTDLNVYLWDVHHSKRLIKVDGVVTKKRKSYCVDFA AIQPQIALLQEMHVTHFRFSLDWALILPLGNQSQVNHTILQYYRCMAS ELVRVNITPVVALWQPMAPNQGLPRLLARQGAWENPYTALAFAEYARL CFQELGHHVKLWITMNEPYTRNMTYSAGHNLLKAHALAWHVYNEKFRH AQNGKISIALQADWIEPACPFSQKDKEVAERVLEFDIGWLAEPIFGSG DYPWVMRDWLNQRNNFLLPYFTEDEKKLIQGTFDFLALSHYTTILVDS EKEDPIKYNDYLEVQEMTDITWLNSPSQVAVVPWGLRKVLNWLKFKYG DLPMYIISNGIDDGLHAEDDQLRVYYMQNYINEALKAHILDGINLCGY FAYSFNDRTAPRFGLYRYAADQFEPKASMKHYRKIIDSNGFPGPETLE RFCPEEFTVCTECSFFHTRKSLLAFIAFLFFASIISLSLIFYYSKKGR RSYK |

### Klotho

Klotho plays important regulatory and protective roles in, *inter alia,* memory loss, stress, synaptic plasticity, biopolar disorder, epilepsy, Alzheimer's disease, Parkinson's disease, multiple sclerosis, myelin-related disease, neurogenic decline, neurodegeneration, amyotrophic lateral sclerosis, cancer, muscle function, cardiovascular function and kidney dysfunction (Vo et al., 2018. Brain Plast. 3: 183-194).

The human Klotho gene is located on chromosome 13 and comprises five exons. The Klotho protein primarily exists in one of three forms. Transmembrane Klotho is an approximately 130 kDa, glyclosylated, Type I transmembrane protein. The transmembrane Klotho can be shed from the cell surface by ADAM10/17 metalloproteinases into a soluble form that is detectable in serum and CSF (Bloch et al., 2009. FEBS Lett. 583(19): 3221-3224; Chen et al., 2007. Proc. Natl Acad. Sci. USA. 104(50): 19796-19801; Matsumura et al., 1998. Biochem. Biophys. Res. Commun. 242(3): 626-630). A third, secreted form of Klotho is generated by alternative splicing of exon 3 to produce a 70 kDa protein which is detectable in blood and CSF (Masso et al., 2015. PLoS One. 10(11): e0143623). The transmembrane (mKL) and soluble forms (KL1, KL2, KL1+KL2, sKL) of Klotho have important functions in many homeostatic processes.

Klotho has been shown to inhibit the IGF-1 and Wnt pathways. Klotho also modulates the activity of bFGF, which activates ERK1/2. Klotho is cleaved into the entire extracellular domain, and also into the subdomains KL1 and KL2. KL1 and the secreted differentially spliced Klotho (s-KL) isoforms inhibit cancer cell growth and inhibit the IGF-1, bFGF and Wnt pathways.

Table 1 lists various Klotho sequences that are relevant to the present disclosure. Those skilled in the art will understand that several different Klotho alleles exist among humans, and all of those alleles are envisaged by the present disclosure. Skilled persons will also understand that greater levels of sequence variation may exist in genomic regions which do not directly encode amino acids. It will also be understood that references herein to Klotho should be understood as including the different isoforms and fragments of Klotho unless context or biology dictate otherwise. The nucleic acid molecules encoding Klotho referred to herein may be derived from human Klotho or non-human (eg, murine) Klotho. In some examples, the Klotho is a KL-VS variant. The KL-VS variant contains two mutations in the coding sequence that result in amino acid substitutions, namely, F352V and C370S.

### Increased body weight

Weight gain is an increase in body weight. This can involve an increase in muscle mass, fat deposits, excess fluids such as water or other factors. Weight gain can be a process of normal and healthy development; it may also be a symptom of a serious medical condition or lead to serious medical conditions. Weight gain occurs when more energy (as calories from food and beverage consumption) is gained than the energy expended by life activities, including normal physiological processes and physical exercise. If enough weight is gained due to increased body fat deposits, one may become overweight or obese, generally defined as having more body fat (adipose tissue) than is considered good for health. The Body Mass Index (BMI) measures body weight in proportion to height, and defines optimal, insufficient, and excessive weight based on the ratio. The BMI Categories are: underweight = <18.5, normal weight = 18.5-24.9, overweight = 25-29.9, and obesity = BMI of 30 or greater. Having excess adipose tissue (fat) is a common condition, especially where food supplies are plentiful and lifestyles are sedentary. Obesity and certain forms of overweight may increase the risk of several diseases, such as diabetes, heart disease, and some cancers, and may lead to short- and long-term health problems during pregnancy. Excess adipose tissue can lead to medical problems; however, a round or large figure (overweight) does not necessarily imply a medical problem, and is sometimes not primarily caused by adipose tissue. If too much weight is gained (resulting in obesity), serious health side-effects may follow. A large number of medical conditions have been associated with obesity. Health consequences are categorised as being the result of either increased fat mass (osteoarthritis, obstructive sleep apnea, social stigma) or increased number of fat cells (diabetes, some forms of cancer, cardiovascular disease, non-alcoholic fatty liver disease). There are alterations in the body's response to insulin (insulin resistance), a proinflammatory state and an increased tendency to thrombosis (prothrombotic state).

### Weight loss

The present invention achieves weight loss by appropriate administration of Klotho nucleic acids to mammals, especially to human individuals. Weight loss or (synonymously) reduction in body weight, in the context of medicine, health, or physical fitness (and in the context of the present invention), refers to a reduction of the total body mass, by a mean loss of fluid, body fat (adipose tissue), or lean mass (namely bone mineral deposits, muscle, tendon, and other connective tissue). Weight loss can either occur unintentionally because of malnourishment or an underlying disease, or from a conscious effort to improve an actual or perceived overweight or obese state. "Unexplained" weight loss that is not caused by reduction in calorific intake or increase in exercise is called cachexia and may be a symptom of a serious medical condition.

Intentional weight loss is the loss of total body mass as a result of efforts to improve fitness and health, or to change appearance through slimming. Weight loss is the main treatment for obesity, and there is substantial evidence this can prevent progression from prediabetes to type 2 diabetes with a 7-10% weight loss and manage cardiometabolic health for diabetic people with a 5-15% weight loss. Weight loss in individuals who are overweight or obese can reduce health risks, increase fitness, and may delay the onset of diabetes. It could reduce pain and increase movement in people with osteoarthritis of the knee. Weight loss may be achieved by adopting a lifestyle in which fewer calories are consumed than are expended. Depression, stress or boredom may contribute to weight increase, and in these cases, individuals are advised to seek medical help. According to the UK National Health Service and the Dietary Guidelines for Americans, those who achieve and manage a healthy weight do so most successfully by being careful to consume just enough calories to meet their needs, and being physically active.

Anti-obesity medication or weight loss medications are pharmacological agents that reduce or control excess body fat. These medications alter one of the fundamental processes of the human body, weight regulation, by reducing appetite and consequently energy intake, increasing energy expenditure, redirecting nutrients from adipose to lean tissue, or interfering with the absorption of calories. Weight loss drugs have been developed since the early twentieth century, and many have been banned or withdrawn from the market due to adverse effects, including deaths; other drugs proved ineffective. Although many earlier drugs were stimulants such as amphetamines, currently GLP-1 agonists are successfully applied to obtain weight loss. The drugs semaglutide, naltrexone/bupropion, liraglutide, and orlistat are FDA approved for long-term weight management. So far, however, no drug has been shown to be as effective at long-term weight reduction as bariatric surgery. The main treatment modalities for obesity remain dieting (healthy diet and caloric restriction) and physical exercise.

Treatments according to the present invention which are successful in reducing body weight (i.e. which are efficient in obtaining a body weight reduction as the result of treatment or which promote loss of body weight by efficient treatment) may be therapeutical or may be non-therapeutical (cosmetic), depending on the weight status of the individual to be treated. As used herein, treatment of individuals (which are otherwise healthy with respect to their body weight) with normal weight or overweight is regarded as being a non-therapeutic treatment whereas the treatment of individuals with obesity is regarded as being a therapeutic treatment. Treatment for reducing body weight on human individuals with a body-mass index of at least 27 with at least one weight-related comorbidity is also regarded as being therapeutical. This patient population is (as patients with obesity) considered to have sufficiently high baseline health risks to justify the use of anti-obesity medication. Landry et al. (Met. Clin. Exp. 121 (2021), 154819) have reviewed how circulating Klotho regulates metabolism via distinct central and peripheral mechanisms. The effects of Klotho on peripheral functions are summarised as being "multifaceted" (i.e. not absolutely clear and sometimes ambiguous). Whereas a reduction of food intake was reported after intracerebroventricular (ICV) of klotho administration in mice, no such effects were reported for peripheral administration, whereas a clear connection with reduction of body weight is present for fibroblast growth factor receptor (FGFR) inhibition with FGF19 and/or FGF 21. Although Klotho has been associated and suggested for a myriad of uses (as also in WO 2017/210607 A1 for Klotho protein produced in CHO cells, US 2021/0371839 A1 for stabilizing Klotho protein, or WO 2020/039425 A1 for Klotho expression vectors for treating cancer), Klotho encoding nucleic acids were not known to be effective for therapeutic and non-therapeutic body weight loss. Klotho-encoding nucleic acid was therefore not disclosed as having a relevant weight-reduction effect. This effect is experimentally shown and proven to be significant by the present invention (see also: example section, below).

Therefore, the present invention provides a non-therapeutic method for obtaining or promoting weight loss in a mammal, especially in a human individual, comprising supplementing a cell of the mammal with a Klotho protein by administering to the cell a nucleic acid encoding the Klotho protein such that the nucleic acid is expressed within the cell to produce the Klotho protein so as to obtain weight loss in said mammal.

According to another aspect, the present invention provides a method of treating a mammal, especially a human individual, with pathologically increased body weight, the method comprising administering to the mammal a therapeutically effective amount of a nucleic acid encoding a Klotho protein such that the nucleic acid is expressed within a cell of the subject to produce the Klotho protein in an efficient amount to obtain weight loss in said mammal. Accordingly, this aspect is also drawn to a nucleic acid encoding a Klotho protein for use for reducing body weight.

### Nucleic acids and nucleic acid modifications

The nucleic acid of the present disclosure may be DNA or RNA. Preferably, the nucleic acid is an RNA molecule (such as mRNA) encoding Klotho or an isoform or fragment thereof (e.g., human). In some examples, the nucleic acid is an RNA molecule encoding the transmembrane isoform of Klotho (m-KL). The m-KL may be between about 130 kDa and 140 kDa. Preferably, the m-KL is about 135 kDa. In some examples, the nucleic acid is an RNA molecule encoding the secreted isoform of Klotho (s-KL). The s-KL may be between about 65 kDa and 75 kDa. Preferably, the s-KL is about 70 kDa. In some examples, the nucleic acid is an RNA molecule encoding a soluble KL1+KL2 or KL1 isoform of Klotho. Amino acid sequences of KL1 can be readily deduced from the nucleic acid sequences set forth in SEQ ID NO. 3 and SEQ ID NO. 7.

In some examples, the nucleic acid encodes a secreted isoform of Klotho comprising the sequence set forth in SEQ ID NO. 12, or a sequence having at least 80% identity, or at least 85% identity, or at least 90% identity, or at least 95% identity, or at least 97%, 98% or 99% identity, to the sequence set forth in SEQ ID NO. 12. In some examples, the nucleic acid encodes a transmembrane isoform of Klotho comprising the sequence set forth in SEQ ID NO. 15, or a sequence having at least 80% identity, or at least 85% identity, or at least 90% identity, or at least 95% identity, or at least 97%, 98% or 99% identity, to the sequence set forth in SEQ ID NO. 15.

In certain examples, the nucleic acid of the present disclosure comprises modifications, for example, end modifications, eg, 5'-end modifications (phosphorylation, conjugation, inverted linkages) or 3'-end modifications (conjugation, DNA nucleotides, inverted linkages, etc.); base modifications, eg, replacement with stabilizing bases, destabilizing bases, or bases that base pair with an expanded repertoire of partners, removal of bases (abasic nucleotides), or conjugated bases; sugar modifications (eg, at the 2'-position or 4'-postion) or replacement of the sugar; and/or backbone modifications, including modification or replacement of the phosphodiester linkages. Modified RNA backbones that do not include a phosphorus atom therein have backbones that are formed by short chain alkyl or cycloalkyl internucleoside linkages, mixed heteroatoms and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH₂ component parts.

### Administration

The delivery of a nucleic acid of the present disclosure to a cell, eg, a cell within a subject, such as a human subject, can be achieved in a number of different ways.

Factors to consider for *in vivo* delivery include biological stability of the delivered nucleic acid, prevention of non-specific effects and accumulation of the nucleic acid in the target tissue. The non-specific effects of the nucleic acid can be minimized by local administration, for example, by direct injection or implantation into a tissue. Modification of the nucleic acid or the pharmaceutical carrier can also permit tissue-specific targeting and reduced off-target effects. Nucleic acid molecules can be modified by chemical conjugation to lipophilic groups such as cholesterol to enhance cellular uptake and to prevent degradation (see, eg, Soutschek, et al. 2004. Nature 432:173-178).

Those skilled in the art will understand that Klotho mRNA may be delivered to a subject or a cell by various means. Suitable delivery methods may include those described in Golombek et al. 2018. Mol. Ther. Nucleic Acids 11: 382-392. In some examples, the nucleic acid may be delivered using drug delivery systems such as a nanoparticle, a dendrimer, a polymer, liposomes, or a cationic delivery system. Cationic delivery systems facilitate binding of a nucleic acid molecule and also enhance interactions at the negatively charged cell membrane to permit efficient uptake of the nucleic acid by the cell. Cationic lipids, dendrimers, or polymers can either be bound to a nucleic acid, or induced to form a vesicle or micelle (see eg, Kim, et al. 2008. J Controlled Release 129(2): 107-116) that encases a nucleic acid. The formation of vesicles or micelles further prevents degradation of the nucleic acid when administered systemically. Methods for making and administering cationic nucleic acid complexes can readily be performed by those skilled in the art (see eg, Sorensen, et al. 2003. J. Mal. Biol. 327:761-766; Verma, et al. 2003. Clin. Cancer Res. 9:1291-1300; Arnold, et al. 2007. J. Hypertens. 25: 197-205). Some non-limiting examples of drug delivery systems useful for systemic delivery of RNA include DOTAP (Sorensen, et al. 2003. J. Mal. Biol. 327:761-766; Verma, et al. 2003. Clin. Cancer Res. 9:1291-1300), oligofectamine, solid nucleic acid lipid particles (Zimmermann, et al. 2006. Nature 441: 111-114), cardiolipin (Chien, et al. 2005. Cancer Gene Ther. 12:321-328; Pal, et al. 2005. Int J. Oneal. 26: 1087-1091), polyethyleneimine (Aigner, 2006. J. Biomed. Biotechnol. 71659), Arg-Gly-Asp (RGD) peptides (Liu, 2006. Mal. Pharm. 3:472-487), and polyamidoamines (Tomalia, et al. 2007. Biochem. Soc. Trans. 35:61-67). In some examples, the nucleic acid forms a complex with cyclodextrin for systemic administration. In some examples, the nucleic acid of the present disclosure is formulated with a lipid nanoparticle composition comprising a cationic lipid/Cholesterol/PEG-C-DMA/DSPC (eg, in a 40/48/2/10 ratio), a cationic lipid/Cholesterol/PEG-DMG/DSPC (eg, in a 40/48/2/10 ratio), or a cationic lipid/Cholesterol/PEG-DMG (eg, in a 60/38/2 ratio). In some examples, the cationic lipid is Octyl CL in DMA, DL in DMA, L-278, DlinKC2DMA or MC3. In certain examples, the nucleic acid is conjugated to, or complexed with, another compound, eg, to facilitate delivery of the nucleic acid (eg, CDM-LBA, CDM-Pip-LBA, CDM-PEG, CDM-NAG etc.). In certain examples, polyethylene glycol (PEG) is covalently attached to the nucleic acid. In further examples, the nucleic acid is formulated or complexed with polyethyleneimine or a derivative thereof, such as polyethyleneimine-polyethyleneglycol-N-acetylgalactosamine (PEI-PEG-GAL) or polyethyleneimine-polyethyleneglycol-tri-N-acetylgalactosamine (PEI-PEG-triGAL) derivatives.

In one aspect, the present disclosure provides the use of a LNP encapsulating an RNA molecule encoding Klotho or an isoform of Klotho for achieving weight loss in a mammal, especially in a human individual. The LNP may comprise: an ionizable cationic lipid; DOPE; cholesterol; and DMG-PEG. In some examples, the LNP comprises: an ionizable cationic lipid; DOPE; cholesterol; and DMG-PEG at a ratio of about 50 (cationic lipid); 10 (DOPE); 38.5 (cholesterol); 1.5 (DMG-PEG). In some examples, the LNP comprises: PIP ionizable cationic lipid; DOPE; cholesterol; and DMG-PEG. In some examples, the LNP comprises: PIP ionizable cationic lipid; DOPE; cholesterol; and DMG-PEG at a ratio of about 50 (PIP ionizable cationic lipid); 10 (DOPE); 38.5 (cholesterol); 1.5 (DMG-PEG). In some examples, the LNP comprises: PIP ionizable cationic lipid; DOPE; cholesterol; and DMG-PEG. In some examples, the LNP comprises: A52P ionizable cationic lipid; DOPE; cholesterol; and DMG-PEG at a ratio of about 50 (A52P ionizable cationic lipid); 10 (DOPE); 38.5 (cholesterol); 1.5 (DMG-PEG). Suitable methods for producing LNPs for encapsulating RNA are also described in An et al. 2017. Cell Reports. 21 :3548-3558; Sabnis et al. 2018. Molecular Therapy. 26(6):1059-1519; Karadagi et al. 2020. Scientific Reports. 10:7052; and US Patent Publication No. US 2017/0210697.

In examples where the nucleic acid of the present disclosure is an RNA molecule, that molecule may be expressed from a DNA vector. Expression can be transient (in the order of hours to weeks) or sustained (weeks to months or longer), depending upon the specific construct used and the target tissue or cell type. Transgenes expressing the RNA can be introduced as a linear construct, a circular plasmid, or a viral vector, which can be an integrating or non-integrating vector. The transgene can also be constructed to permit inheritance as an extrachromosomal plasmid (Gassmann, et al. 1995. Proc. Natl. Acad. Sci. USA 92:1292).

RNA expression vectors are generally DNA plasmids or viral vectors. Expression vectors compatible with eukaryotic cells, preferably those compatible with vertebrate cells, can be used to produce recombinant constructs for the expression of a RNA molecule as described herein. Classes of viral systems that are used in gene therapy can be categorized into two groups according to whether their genomes integrate into host cellular chromatin (oncoretroviruses and lentiviruses) or persist in the cell nucleus predominantly as extrachromosomal episomes (adeno-associated virus, adenoviruses and herpesviruses). In certain examples, the viral vector is an adenoviral (AdV) vector. Adenoviruses are medium sized double-stranded, non-enveloped DNA viruses with linear genomes that are between 26-48 Kbp. In other examples, the viral vector is from the Parvoviridae family. The Parvoviridae is a family of small single-stranded, non-enveloped DNA viruses with genomes approximately 5000 nucleotides long. Included among the family members is adeno-associated virus (AAV). In some examples, the viral vector of the present disclosure is an AAV. In other examples, the viral vector is from the family Retroviridae. Retroviruses comprise single-stranded RNA animal viruses that are characterized by two unique features. First, the genome of a retrovirus is diploid. Second, this RNA is transcribed by the virion-associated enzyme reverse transcriptase into double-stranded DNA. This dsDNA or provirus can then integrate into the host genome and be passed from parent cell to progeny cells as a stably-integrated component of the host genome. In certain examples, the viral vector is a lentivirus. Lentivirus vectors are often pseudotyped with vesicular steatites virus glycoprotein (VSV-G), and have been derived from the human immunodeficiency virus (HIV); visan-maedi, which causes encephalitis (visna) or pneumonia in sheep; equine infectious anemia virus (EIAV), which causes autoimmune hemolytic anemia and encephalopathy in horses; feline immunodeficiency virus (FIV), which causes immune deficiency in cats; bovine immunodeficiency virus (BIV) which causes lymphadenopathy and lymphocytosis in cattle; and simian immunodeficiency virus (SIV), which causes immune deficiency and encephalopathy in non-human primates. A lentiviral-based construct used to express RNA of the disclosure preferably comprises sequences from the 5' and 3' long terminal repeats (LTRs) of a lentivirus. In some examples, the viral construct comprises an inactivated or self-inactivating 3' LTR from a lentivirus. The 3' LTR may be made self-inactivating by any method known in the art. Viral vector systems which can be used in the methods and compositions described herein include, but are not limited to, (a) adenovirus vectors; (b) retrovirus vectors, including but not limited to lentiviral vectors, 29yrroli murine leukemia virus, etc.; (c) adeno-associated virus vectors; (d) herpes simplex virus vectors; I SV 40 vectors; (f) polyoma virus vectors; (g) papilloma virus vectors; (h) picornavirus vectors; (i) pox virus vectors such as an orthopox, eg, vaccinia virus vectors or avipox, eg, canary pox or fowl pox; and (j) a helper-dependent or gutless adenovirus.

In some examples, the nucleic acid of the present disclosure is administered via a route such as enteral (into the intestine), gastroenteral, epidural (into the dura matter), oral (by way of the mouth), transdermal, peridural, intracerebral (into the cerebrum), intracerebroventricular (into the cerebral ventricles), epicutaneous (application onto the skin), intradermal, (into the skin itself), subcutaneous (under the skin), nasal administration (through the nose), intravenous (into a vein), intravenous bolus, intravenous drip, intraarterial (into an artery), intramuscular (into a muscle), intracardiac (into the heart), intraosseous infusion (into the bone marrow), intraperitoneal, (infusion or injection into the peritoneum), intravesical infusion, intravitreal, (through the eye), intracavernous injection (into a pathologic cavity) intracavitary (into the base of the penis), intravaginal administration, intrauterine, extra-amniotic administration, transdermal (diffusion through the intact skin for systemic distribution), transmucosal (diffusion through a mucous membrane), transvaginal, insufflation (snorting), sublingual, sublabial, enema, eye drops (onto the conjunctiva), in ear drops, auricular (in or by way of the ear), buccal (directed toward the cheek), conjunctival, cutaneous, dental (to a tooth or teeth), electro-osmosis, endocervical, endosinusial, endotracheal, extracorporeal, hemodialysis, infiltration, interstitial, intra-abdominal, intra-amniotic, intra-articular, intrabiliary, intrabronchial, intrabursal, intracartilaginous (within a cartilage), intracaudal (within the cauda equine), intracorneal (within the cornea), dental intracornal, intracoronary (within the coronary arteries), intracorporus cavernosum (within the dilatable spaces of the corporus cavernosa of the penis), intradiscal (within a disc), intraductal (within a duct of a gland), intraduodenal (within the duodenum), intradural (within or beneath the dura), intraepidermal (to the epidermis), intraesophageal (to the esophagus), intragastric (within the stomach), intragingival (within the gingivae), intraileal (within the distal portion of the small intestine), intralesional (within or introduced directly to a localized lesion), intraluminal (within a lumen of a tube), intralymphatic (within the lymph), intramedullary (within the marrow cavity of a bone), intrameningeal (within the meninges), intramyocardial (within the myocardium), intraocular (within the eye), intraovarian (within the ovary), intrapericardial (within the pericardium), intrapleural (within the pleura), intraprostatic (within the prostate gland), intrapulmonary (within the lungs or its bronchi), intrasinal (within the nasal or periorbital sinuses), intraspinal (within the vertebral column), intrasynovial (within the synovial cavity of a joint), intratendinous (within a tendon), intratesticular (within the testicle), , intrathoracic (within the thorax), intratubular (within the tubules of an organ), intratumor (within a tumor), intratympanic (within the aurus media), intravascular (within a vessel or vessels), intraventricular (within a ventricle), iontophoresis (by means of electric current where ions of soluble salts migrate into the tissues of the body), irrigation (to bathe or flush open wounds or body cavities), laryngeal (directly upon the larynx), nasogastric (through the nose and into the stomach), occlusive dressing technique (topical route administration, which is then covered by a dressing that occludes the area), ophthalmic (to the external eye), oropharyngeal (directly to the mouth and pharynx), parenteral, percutaneous, periarticular, periodontal, rectal, respiratory (within the respiratory tract by inhaling orally or nasally for local or systemic effect), retrobulbar (behind the pons or behind the eyeball), intramyocardial (entering the myocardium), soft tissue, subarachnoid, subconjunctival, submucosal, topical, transplacental (through or across the placenta), transtracheal (through the wall of the trachea), transtympanic (across or through the tympanic cavity), ureteral (to the ureter), urethral (to the urethra), vaginal, caudal block, biliary perfusion, cardiac perfusion, photopheresis and spinal. Routes of administration will also be dependent on whether the aim is to obtain or provide a therapeutic treatment, which may - depending on the medical need for reduction of body weight - include more aggressive and invasive methods for administration, or a non-therapeutic treatment which may be mostly done by non- or less invasive methods, such as oral, peripheral, mucosal, topical, subcutaneous or intramuscular administration.

Modes of administration include injection, infusion, instillation, and/or ingestion" "Inject"on" includes, without limitation, intravenous, intramuscular, intra-arterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, and intrasternal injection and infusion. Preferably, the nucleic acid of the present disclosure is administered intravenously, intramuscularly or intraperitoneally.

In addition, components may be formulated to permit release over a prolonged period of time. A release system can include a matrix of a biodegradable material or a material which releases the incorporated components by diffusion. The components can be homogeneously or heterogeneously distributed within the release system. A variety of release systems may be useful, however, the choice of the appropriate system will depend upon the rate of release required by a particular application. Both non-degradable and degradable release systems can be used. Suitable release systems include polymers and polymeric matrices, non-polymeric matrices, or inorganic and organic excipients and diluents such as, but not limited to, calcium carbonate and sugar (for example, trehalose). The release system material can be selected so that components having different molecular weights are released by diffusion or through degradation of the material. Representative synthetic, biodegradable polymers include, for example: polyamides such as poly(amino acids) and poly(peptides); polyesters such as poly(lactic acid), poly(glycolic acid), poly(lactic-co-glycolic acid), and poly(caprolactone); poly(anhydrides); polyorthoesters; polycarbonates; and chemical derivatives thereof (substitutions, additions of chemical groups, for example, alkyl, alkylene, hydroxylations, oxidations, and other modifications routinely made by those skilled in the art), copolymers and mixtures thereof. Representative synthetic, non-degradable polymers include, for example: polyethers such as polyethylene oxide), polyethylene glycol), and poly(tetramethylene oxide); vinyl polymers-polyacrylates and polymethacrylates such as methyl, ethyl, other alkyl, hydroxyethyl methacrylate, acrylic and methacrylic acids, and others such as poly(vinyl alcohol), poly(vin32yrrolidoneone), and poly(vinyl acetate); poly(urethanes); cellulose and its derivatives such as alkyl, hydroxyalkyl, ethers, esters, nitrocellulose, and various cellulose acetates; polysiloxanes; and any chemical derivatives thereof (substitutions, additions of chemical groups, for example, alkyl, alkylene, hydroxylations, oxidations, and other modifications routinely made by those skilled in the art), copolymers and mixtures thereof. Poly(lactide-co-glycolide) microspheres can also be used.

The nucleic acids of the present disclosure may be administered as a composition which includes materials for increasing the biological stability of the nucleic acid and/or materials that increase the ability of the composition to penetrate a particular cell type. The nucleic acid is preferably administered with a pharmaceutically acceptable carrier (eg, physiological saline), which is selected on the basis of the mode and route of administration, and standard pharmaceutical practice. In some examples, an isotonic formulation is used. Generally, additives for isotonicity can include sodium chloride, dextrose, mannitol, sorbitol and lactose. In some cases, isotonic solutions such as phosphate buffered saline are preferred. Stabilizers include gelatin and albumin. In some examples, a vasoconstriction agent is added to the formulation. The compositions according to the present disclosure are preferably sterile and pyrogen free.

Dosages may vary with the type and severity of the condition to be treated, and may include single or multiple dosses. Specific dosage regimens may be adjusted over time according to the individual need and the professional judgment of the practitioner administering the composition. When administered to a human subject, the dosage regimen may vary depending on a variety of factors including the type and severity of the condition, the age, sex, weight or medical condition of the subject connected with the aim to reduce body weight and the route of administration. A Klotho blood test may be used to determine an appropriate dose until a desired level of Klotho protein in blood is reached.

Compositions comprising the nucleic acid described herein may be administered over a period of hours, days, weeks, or months, depending on several factors, including the type and severity of the condition being treated, whether a recurrence is considered likely, etc. The administration may be constant, eg, constant infusion over a period of hours, days, weeks, months, etc. Alternatively, the administration may be intermittent, eg, once per day over a period of days, once per hour over a period of hours, or any other such schedule as deemed suitable. For example, in the case of obesity, the nucleic acid of the present disclosure is preferably administered once, twice or three times per week of a period of one week, or two weeks, or three weeks, or four weeks, or five weeks, or six weeks, or seven weeks or eight weeks or longer.

### Treatments

The present disclosure provides a method of treating a Klotho-related disease or disorder in a subject comprising administering to the subject a therapeutically effective amount of a nucleic acid encoding Klotho, an isoform of Klotho or a fragment of Klotho. Klotho-related diseases are typically those which are associated with low levels of Klotho. For example, a patient may have a blood Klotho level which is lower than that of a healthy subject. The patient may have Klotho levels that are lower than a threshold value which is determined based upon the Klotho levll in a healthy subject or a population of healthy subjects. In some examples, disease progression may be associated with reduced Klotho levels over time. The disease or disorder to be therapeutically treated in connection to increased body weight may be obesity, cancer (such as pancreatic cancer), acute kidney injury or chronic kidney disease, a neurological disorder, Duchenne muscular dystrophy, cardiovascular disease, cognitive impairment, Alzheimer's disease, renal dysfunction, multiple sclerosis, Parkinson's disease, amyotrophic lateral sclerosis, acute kidney injury or chronic kidney disease. Individuals may be healthy, aged subjects with low blood Klotho levels. In some examples, the nucleic acid encoding Klotho is administered in combination with an agent that increases endogenous Klotho production. Suitable agents may include those described in International Patent Application numbers PCT/US2018/063837, PCT/US2019/049918 and PCT/US2019/066535. In some examples, the nucleic acid encoding Klotho is administered in combination with a non-invasive method of increasing endogenous Klotho production such as bioelectric stimulation.

The neurological disorder may be associated with memory loss, psychological dysfunction, stress, biopolar disorder, epilepsy, dementia (eg, post stroke dementia, post-traumatic dementia, senile dementia), Alzheimer's disease, Parkinson's disease, Huntington's disease, Creutzfeldt-Jakob disease, ataxia telangiectasia, craniocerebral trauma, amyotrophic lateral sclerosis (ALS), depression, schizophrenia, multiple sclerosis, myelin-related disease, oxidative stress, neurogenic decline or neurodegeneration. Symptoms of neurological disorders may include cognition impairment, memory loss, anxiety, depression, insomnia, disorientation, irrational fear, decline of motor skills or locomotor activity, neophobia, apathy, agitation, tremors, loss of balance, irritability or agoraphobia.

The method may further comprise administering to the subject an active agent suitable for the treatment of a neurological disorder such as donepezil hydrochloride, memantine, rivastigmine, ligustilide, aripiprazole, asenapine, cariprazine, clozapine, lurasidone, olanzapine, quetiapine, risperidone, ziprasidone, xenazine, tetrabenazine, baclofen, lioresal, kemstro, deutetrabenazine, austedo, cannabis extract, a cannabinoid or cannabinol, an antidepressant, a cholinesterase inhibitor, an antipsychotic, antioxidants, levodopa, carbidopa, trazodone or dibenzoylmethane. Those skilled in the art will be aware of other active agents that may be suitable for treatment of neurological disorders.

The present disclosure provides method of treating obesity in a subject the method comprising administering the subject a therapeutically effective amount of a nucleic acid encoding Klotho, an isoform of Klotho or a fragment of Klotho. The nucleic acid may be administered in combination with further agents known to have a body weight reducing effect, such as semaglutide, phentermine/topiramate, naltrexone/bupropion, liraglutide, gelesis100, orlistad, methamphetamine, and/or tirzepatideʺ‴.

The present disclosure also provides methods for treating an individual with increased body weight further having an age-related condition comprising administering the subject a therapeutically effective amount of a nucleic acid encoding Klotho, an isoform of Klotho or a fragment of Klotho. The age-related condition may be sarcopenia, skin atrophy, muscle wasting, brain atrophy, atherosclerosis, arteriosclerosis, pulmonary emphysema, osteoporosis, osteoarthritis, immunologic incompetence, high blood pressure, dementia, Huntin'ton's disease, Alzhe'mer's disease, cataracts, age-related macular degeneration, prostate cancer, stroke, memory loss, wrinkles, impaired kidney function or hearing loss.

The present disclosure also provides methods for treating an individual with increased body weight further having a muscular disorder such as muscle atrophy and muscular dystrophy (eg, Duchene muscular dystrophy) the method comprising administering the subject a therapeutically effective amount of a nucleic acid encoding Klotho, an isoform of Klotho or a fragment of Klotho.

The present disclosure also provides methods for treating an individual with increased body weight further having a metabolic disorder the method comprising administering the subject a therapeutically effective amount of a nucleic acid encoding Klotho, an isoform of Klotho or a fragment of Klotho. In certain examples, the metabolic disorder is selected from Type II Diabetes, Metabolic Syndrome, hyperglycemia and obesity.

### Clauses of the disclosure

Set forth below are non-limiting methods and compositions of the present disclosure.

Clause 1. A non-therapeutic method for obtaining or promoting weight loss in a mammal, especially in a human individual, comprising supplementing a cell of the mammal with a Klotho protein by administering to the cell a nucleic acid encoding the Klotho protein such that the nucleic acid is expressed within the cell to produce the Klotho protein so as to obtain weight loss in said mammal.

Clause 2. A method of treating a mammal, especially a human individual, with pathologically increased body weight, the method comprising administering to the mammal a therapeutically effective amount of a nucleic acid encoding a Klotho protein such that the nucleic acid is expressed within a cell of the subject to produce the Klotho protein in an efficient amount to obtain weight loss in said mammal.

Clause 3. The method of clause 1 or clause 2 wherein the nucleic acid is administered in combination with further agents known to have a body weight reducing effect, preferably semaglutide, phentermine/topiramate, naltrexone/bupropion, liraglutide, gelesis100, orlistad, methamphetamine, and/or tirzepatide.

Clause 4. The method of clause 2 wherein the individual suffers from obesity.

Clause 5. The method of clause 2 wherein the individual further suffers from acute kidney injury or chronic kidney disease.

Clause 6. The method of clause 2 wherein the individual further suffers from cancer, Duchenne muscular dystrophy, Alzheimer's disease, cognitive impairment, multiple sclerosis, Parkinson's disease or amyotrophic lateral sclerosis.

Clause 7. The method of clause 2 wherein the individual further suffers from pancreatic cancer.

Clause 8. The method of any one of clauses 1 to 7 wherein the nucleic acid is a RNA.

Clause 9. The method of any one of clauses 1 to 8 wherein the nucleic acid is administered in a lipid nanoparticle.

Clause 10. The method of clause 9 wherein the lipid nanoparticle comprises an ionizable cationic lipid, DOPE, cholesterol and DMG-PEG

Clause 11. The method of any one of clauses 1 to 10 wherein the nucleic acid is administered intravenously, intramuscularly, intranasally or intraperitoneally.

Clause 12. The method of any one of clauses 1 to 11 wherein the nucleic acid is administered at least once per week for at least 2 weeks.

Clause 13. The method of any one of clauses 1 to 12 wherein the nucleic acid is administered at least twice per week for at least 2 weeks.

Clause 14. The method of any one of clauses 1 to 13 wherein the nucleic acid encodes a transmembrane isoform of Klotho (m-KL) between about 130 kDa and 140 kDa.

Clause 15. The method of any one of clauses 1 to 13 wherein the nucleic acid encodes a soluble KL1+KL2 isoform of Klotho between about 125 kDa and 135 kDa.

Clause 16. The method of any one of clauses 1 to 13 wherein the nucleic acid encodes a secreted isoform of Klotho (s-KL) between about 65 kDa and 75 kDa.

Clause 17. The method of any one of clauses 1 to 13 wherein the nucleic acid encodes a KL1 isoform of Klotho between about 65 kDa and 75 kDa.

Clause 18. The method of any one of clauses 1 to 17 wherein the Klotho is a KL-VS variant.

Clause 19. The method of any one of clauses 1 to 18 wherein the nucleic acid does not integrate into the genome of the cell.

Clause 20. The method of any one of clauses 1 to 19 wherein the nucleic acid is administered in combination with an agent that increases endogenous Klotho expression.

### Examples

### A. LNP-delivered Klotho

RNA encoding a soluble Klotho isoform (KL1+KL2) and a transmembrane Klotho isoform (m-KL) were tagged to HA. The mRNA sequence of the m-KL used in this study is set forth in SEQ ID NO. 10, and the mRNA sequence of the KL1+KL1 isoform is set forth in SEQ ID NO. 11 (Table 1).

The sequences are based on mouse Klotho but optimised for human codon usage. In the present study, KL1+KL2 is also referred to as soluble Klotho or sol-Klotho. The polyA tail of each RNA is about 60 adenosines (30 adenosines added). UTRs from the mouse beta globin transcript were used.

The RNA was produced from a plasmid, and is equivalent to mRNA produced by TriLink Option 1 with phosphatase treatment and Cap/5'-/3' sequences, co-transcriptional capping with CleanCap and Me-pseudo-UTP. Anti-reverse cap analogs (ARCA) based in vitro transcription was initiated using standard amounts of ATP, CTP, and N1-Methyl-Ψ and a quarter of the amount of GTP, with an abundance of the cap analog ARCA to increase the percentage of capped mRNA strands. An hour after initiation, additional GTP was added to enable further synthesis of mRNA. Four hours after initiation, the process was stopped by adding DNAse to degrade the DNA template and then the remaining RNA was isolated from the reaction components using the Qiagen RNeasy kit.

The RNA molecules were packaged into LNPs. LNPs were prepared using the microfluidic micro mixture device made by Precision NanoSystems (Vancouver, BC). Lipid mixture ratios used were: Ionizable cationic lipid / DOPE / Chol / DMG-PEG = 50/10/38.5/1.5. Injected into the mixture device: one volume of the above lipid mixture in ethanol and three volumes of acetate buffer (0.5 M) containing 200 mg of mRNA. The resultant LNPs were dialyzed against PBS (pH 7.4) for 16 hours to remove ethanol. After overnight dialysis, the LNPs were concentrated with an Amicon-tube to the final concentration. Table 2 shows test results of the LNPs prior to injection.

**Table 2**

| **DLS measurments** | 13.9.21 | | 13.9.21 after dialysis | |
|---|---|---|---|---|
| Group | Z-Ave (d.nm) | PDI | Z-Ave (d.nm) | PDI |
| | 58.54 | 0.069 | 74 | 0.103 |
| formulation 1 -TM-klotho | 59.85 | 0.067 | 73.84 | 0.108 |
| | 60.33 | 0.086 | 73.86 | 0.084 |
| AVERAGE | 59.57 | 0.07 | 73.9 | 0.10 |
| | 57.58 | 0.083 | 76.93 | 0.068 |
| formulation 1 sol-klotho | 60.25 | 0.088 | 76.6 | 0.073 |
| | 61.94 | 0.095 | 76.83 | 0.083 |
| AVERAGE | 59.92 | 0.09 | 76.79 | 0.07 |
| | | | | |

| **Zeta potential measurments** | 28.6.21 | | | |
|---|---|---|---|---|
| Group | Zeta (mV) | | | |
| | 4.27 | | | |
| formulation 1 -TM-klotho | 3.9 | | | |
| | 5.09 | | | |
| AVERAGE | 4.42 | | | |
| | 4.4 | | | |
| formulation 1 sol-klotho | 3.68 | | | |
| | 3.83 | | | |
| AVERAGE | 3.97 | | | |

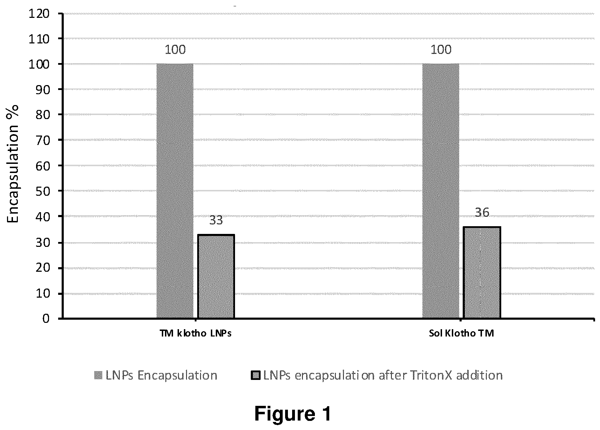

Figure 1 shows the encapsulation percentage of the LNPs. Addition of 2.5% Triton-X into the formulated LNPs was found to cause release of -60% of the RNA.

The LNPs were administered to male C57BI mice aged 10 weeks. Mice were provided an *ad libitum* commercial rodent diet, and free access to drinking water. The mice were acclimatized for at least 5 days. Mice were confined in a limited access facility with environmentally controlled housing conditions throughout the study period, and maintained in accordance with approved standard operating procedures. Automatically controlled environmental conditions were set to maintain temperature at 20-24º C with a relative humidity (RH) of 30-70%, a 12-hr light/12-hr dark cycle and 15-30 air changes/hr in the study room. Temperature, RH and the light cycle were monitored by the control.

200 µL of Klotho mRNA LNP was injected intravenously into the tail vein. Some mice were sacrificed after 6 hours of injection and the rest after 24 hours. Euthanasia was by cervical dislocation. PBS injected to the heart for perfusion. Tissue was surgically removed and frozen in liquid nitrogen.

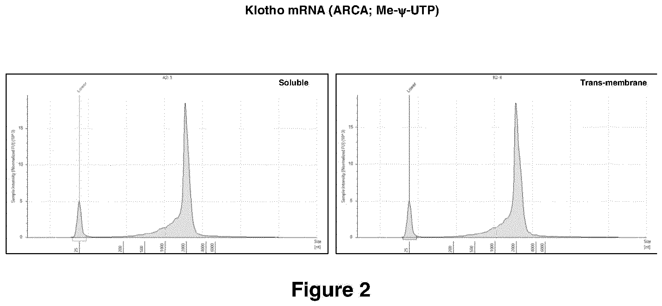

Prior to LNP encapsulation and injection into mice, mRNA produced by *in vitro* transcription was sent to Weizmann Institute for automated electrophoresis (Tapestation) analysis of RNA quality. As shown in Figure 2, both soluble KL and transmembrane KL were readily detectable.

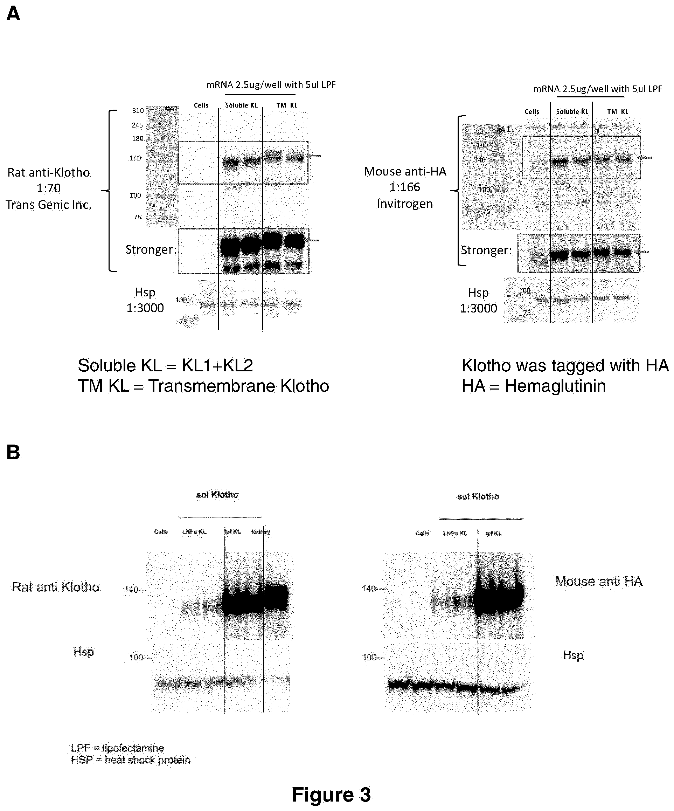

Figure 3A and Figure 3B shows Klotho protein production in HEK293T cells after transfection with Klotho mRNA LNPs. Cells were transfected with 2.5 µg Klotho mRNA in a 12-well plate and collected after 24 hours. Cells were lysed and analysed by 3-8% Tris acetate gel 90 Volt for 3 h. Gels were transferred to nitrocellulose membrane 90 Volt, 1.5 h, then blocked in a blocking solution for 1 h and incubated overnight with primary anti human Klotho monoclonal antibody (Transgene K0603 1:70) or with primary anti mouse anti HA tag monoclonal antibody (abcm 181818). Membrane was washed in Tris buffer saline with Tween 20 (TBST) and incubated with secondary antibody, (1:10,000) for 1 h followed by washing. Finally, A substrate was added to the membranes for enhanced chemiluminescence (ECL) (Pierce^{™} 32109), and the membranes were photographed by the imaging system G-box (Syngene). Heat Shock Protein (HSP 90) was used as internal loading control. Klotho protein was detected by two different methods: (i) Klotho antibody, (ii) HA tag antibody. Cells that were not transfected with Klotho mRNA did not have detectable Klotho. Western Blots clearly show the two Klotho isoforms.

Experiments were conducted on C57 mice as summarised in Table 3. The study included 3 experimental groups with 6 mice in each group. Animals from experimental group 1 were treated with one I.V. injection of PBS, in the tail vein, in a total of 200 µl volume. Animals from experimental group 2-3 were treated with one I.V. injection, in the tail vein with the Klotho mRNA LNPs formulations in a total of 200 µl volume. Blood serum (without EDTA) was drawn after 6 h and 24 h, left for 1 h at RT, then centrifuged at 1000G for 10 min, and the supernatant was stored at -20°C until analysed for serum Klotho. All mice were sacrificed after 24 h and liver, kidney, 2 quadricep and 2 gastrocnemius muscles were surgically removed from each animal, immediately frozen in liquid nitrogen, placed in Eppendorf tubes and stored at -80°C until analysis for the presence of Klotho.

**Table 3**

| **Group** | **Injection material** | **Injected Cone. (∞g)** | **Injected Volume (∞l)** | **Injection Schedule** | **Route** | **Blood collection** | **Termination time points** | **Group Size** |
|---|---|---|---|---|---|---|---|---|
| 1 | **PBS** | X | 200ul | **1 injection** | **IV** | **After 6h** | **24h** collection **13.10.2**1 | **6** |
| 2 | **LNPs-TM-Klotho- mRNA** | 100ug | 200ul | | | | | **6** |
| | | | | **12.10.21 x** | | **After 24h** | | |
| 3 | **LNPs-Sol-Klotho- mRNA** | 100ug | 200ul | | | | | **6** |

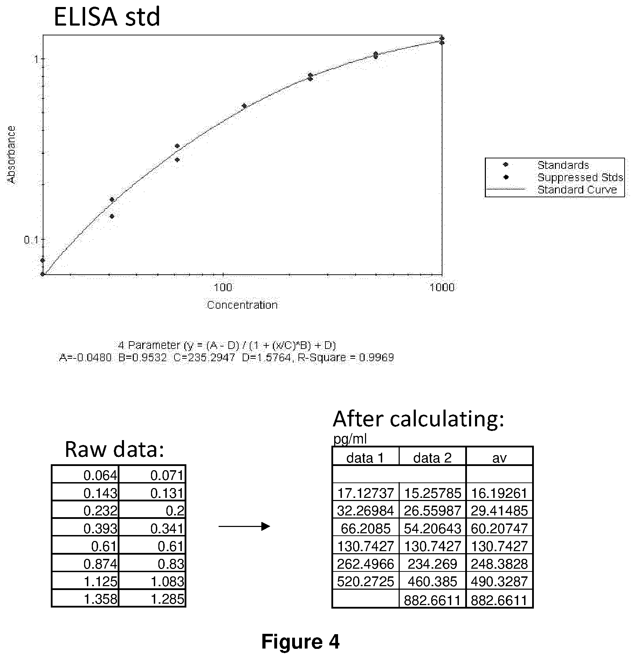

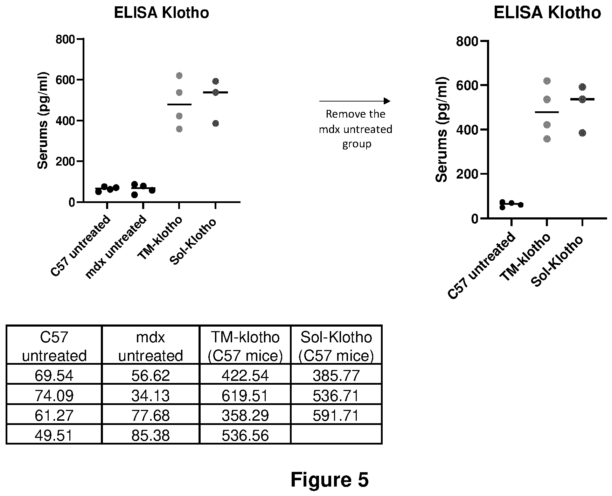

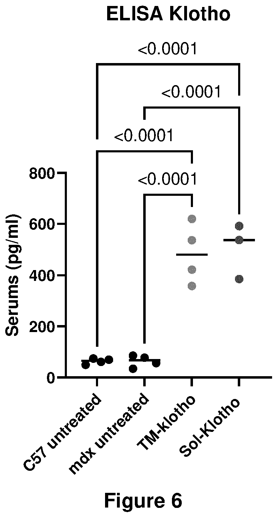

ELISA assays were conducted using an IBL kit for mouse Klotho. Figure 4 shows the standard curve of the ELISA that was used to measure Klotho in mouse serum. As shown in Figure 5, treated C57 mice had much higher serum Klotho protein levels compared to untreated mice (LNP Formulation 1 as described above was used in this study). Figure 6 is a one-way ANOVA showing that the difference was statistically significant.

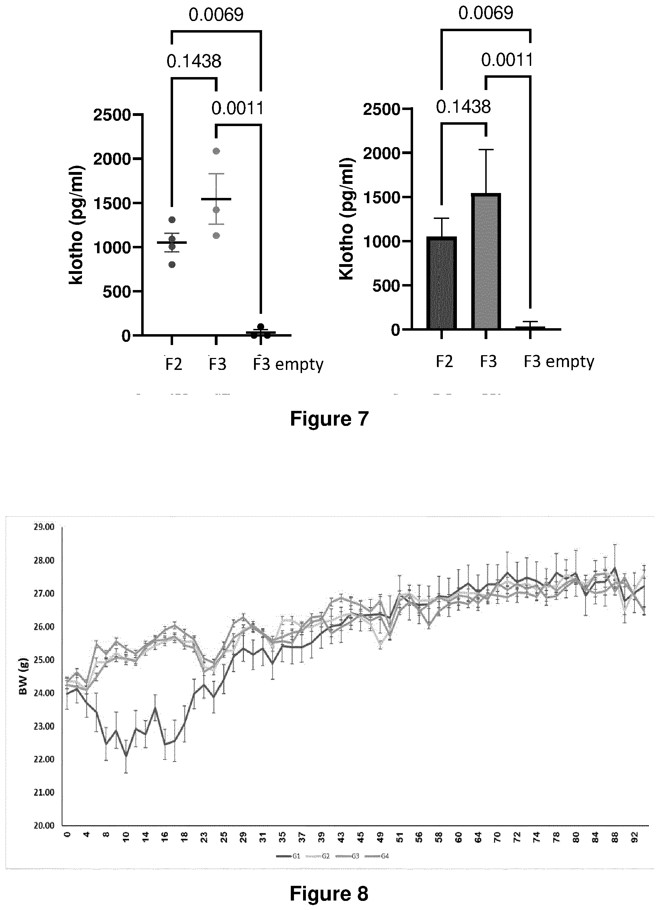

A similar study was conducted using modified LNP formulations, referred to as Formulation 2 and Formulation 3, prepared as follows. The RNA molecules were packaged into LNPs. The LNPs were prepared using the microfluidic micro mixture device made by Precision NanoSystems (Vancouver, BC). Lipid mixture ratios used were as follows: PIP ionizable cationic lipid / DOPE / Chol / DMG-PEG = 50/10/38.5/1.5 in Formulation 2, and A52P ionizable cationic lipid / DOPE / Chol / DMG-PEG = 50/10/38.5/1.5 in Formulations 3. Injected into the mixture device: one volume of the above lipid mixture in ethanol and three volumes of acetate buffer (0.5 M) containing 200 mg of mRNA. The resultant LNPs were dialyzed against PBS (pH 7.4) for 16 hours to remove ethanol. After overnight dialysis, the LNPs were concentrated with an Amicon-tube to the final concentration. Table 4 shows the study plan, and Table 5 shows the particle sizes. Figure 7 shows higher serum levels of Klotho when using Formulation 2 and Formulation 3.

**Table 4**

| **Group** | **Injection material** | **Injected Cone. (∞g)** | **Injected Volume (∞l)** | **Injection Schedule** | **Route** | **Blood collection and termination** | **Group Size** |
|---|---|---|---|---|---|---|---|
| 1 | Formulation - 2 | 100ug | 200ul | **1 injection** | **I.V** | **After 6h from injection** | **4** |
| | **Sol-Klotho-mRNA** | | | | | | |
| 2 | Formulation - 3 | 100ug | 200ul | | | | **3** |
| | **Sol-Klotho-mRNA** | | | **20.9.22** | | **20.9.22** | |
| 3 | **Empty-LNPs** | 100ug | 200ul | | | | **3** |

**Table 5**

| **DLS measurments** | 18.9.22 | | 19.9.22 | |
|---|---|---|---|---|
| Group | Z-Ave (d.nm) | PDI | Z-Ave (d.nm) | PDI |
| Formulation - 2 | 53.66 | 0.078 | 60.05 | 0.089 |
| | 53.83 | 0.101 | 59.41 | 0.091 |
| | 54.31 | 0.07 | 59.4 | 0.092 |
| AVERAGE | 53.93 | 0.08 | 59.62 | 0.09 |
| Formulation - 3 | 54.69 | 0.046 | 72.76 | 0.108 |
| | 55.7 | 0.049 | 73.65 | 0.096 |
| | 56.34 | 0.041 | 72.72 | 0.105 |
| AVERAGE | 55.58 | 0.05 | 73.04 | 0.10 |
| Formulation - 3 empty | 41.3 | 0.086 | 62.59 | 0.067 |
| | 42.28 | 0.08 | 62.24 | 0.071 |
| | 42.35 | 0.085 | 61.56 | 0.089 |
| AVERAGE | 41.98 | 0.08 | 62.13 | 0.08 |
| | | | | |

| **Zeta potential measurments** | 19.8.22 after dyalisis | | | |
|---|---|---|---|---|
| Group | Zeta (mV) | | | |
| Formulation - 2 | 6.61 | | | |
| | 6.72 | | | |
| | 6.67 | | | |
| AVERAGE | 6.7 | | | |
| Formulation - 3 | 3.76 | | | |
| | 4.02 | | | |
| | 3.76 | | | |
| AVERAGE | 3.8 | | | |
| Formulation - 3 empty | -0.0491 | | | |
| | -0.134 | | | |
| | 0.918 | | | |
| AVERAGE | 0.2 | | | |

### B. Reducing body weight in a mouse model

Klotho RNA molecules were packaged into LNPs as described in section A above using Formulation 3. The murine HA-tagged KL1+KL2 isoform described in section A above was used. Three groups (1, 2, 3) had surgery in which blood flow was obstructed to the kidney for 30 minutes, after which it was re-established. The fourth group (G4) had no surgery and no treatment. G1 got Klotho mRNA with LNPs according to the present invention. Klotho mRNA dose was 100 µg. G2 got empty LNPs without Klotho mRNA. G3 and G4 were not treated. For each group, there were a total of 6 injections in a 14-day period (3 injections per week). The injections were made into the tail vein of the mouse.

As shown in Figure 8, body weight was significantly reduced in mice injected with Klotho RNA LNPs compared to control mice and the mice which received empty LNPs. These data of the animal test confirm that the compositions according to the present invention achieve a strong and significant transient weight loss which is not due to the LNPs (because the empty LNPs do not show this effect) but is due to the Klotho nucleic acid.

It will be appreciated by those skilled in the art that the present disclosure may be embodied in many other forms.

## Claims

1. A non-therapeutic method for obtaining or promoting weight loss in a mammal, especially in a human individual, comprising supplementing a cell of the mammal with a Klotho protein by administering to the cell a nucleic acid encoding the Klotho protein such that the nucleic acid is expressed within the cell to produce the Klotho protein so as to obtain weight loss in said mammal.

2. A nucleic acid encoding a Klotho protein for use in a method of treating a mammal, especially a human individual, with pathologically increased body weight, the method comprising administering to the mammal a therapeutically effective amount of a nucleic acid encoding a Klotho protein such that the nucleic acid is expressed within a cell of the subject to produce the Klotho protein in an efficient amount to obtain or promote weight loss in said mammal; or a nucleic acid encoding a Klotho protein for use for reducing body weight.

3. The method of claim 1 or the nucleic acid encoding a Klotho protein for use of claim 2, wherein the nucleic acid is administered in combination with further agents known to have a body weight reducing effect, preferably semaglutide, phentermine/topiramate, naltrexone/bupropion, liraglutide, gelesis100, orlistad, methamphetamine, and/or tirzepatide.

4. The nucleic acid encoding a Klotho protein for use of claim 2, wherein the individual suffers from obesity.

5. The nucleic acid encoding a Klotho protein for use of claim 2, wherein the individual further suffers from acute kidney injury or chronic kidney disease.

6. The nucleic acid encoding a Klotho protein for use of claim 2, wherein the individual further suffers from cancer, Duchenne muscular dystrophy, Alzheimer's disease, cognitive impairment, multiple sclerosis, Parkinson's disease or amyotrophic lateral sclerosis.

7. The nucleic acid encoding a Klotho protein for use of claim 2, wherein the individual further suffers from pancreatic cancer.

8. The method of claim 1 or the nucleic acid encoding a Klotho protein for use of any one of claims 2 to 7, wherein the nucleic acid is a RNA, optionally the RNA is mRNA.

9. The method of claim 1 or 8 or the nucleic acid encoding a Klotho protein for use of any one of claims 2 to 8, wherein the nucleic acid is administered in a lipid nanoparticle, preferably wherein the lipid nanoparticle comprises an ionizable cationic lipid, DOPE, cholesterol and DMG-PEG.

10. The method of any one of claims 1, 8 and 9 or the nucleic acid encoding a Klotho protein for use of any one of claims 2 to 9, wherein the nucleic acid is administered intravenously, intramuscularly, intranasally or intraperitoneally, preferably wherein the nucleic acid is administered at least once per week for at least 2 weeks, especially wherein the nucleic acid is administered at least twice per week for at least 2 weeks.

11. The method of any one of claims 1 and 8 to 10 or the nucleic acid encoding a Klotho protein for use of any one of claims 1 to 13, wherein the nucleic acid encodes a transmembrane isoform of Klotho (m-KL) between about 130 kDa and 140 kDa or a soluble KL1+KL2 isoform of Klotho between about 125 kDa and 135 kDa or a secreted isoform of Klotho (s-KL) between about 65 kDa and 75 kDa or a KL1 isoform of Klotho between about 65 kDa and 75 kDa.

12. The method of any one of claims 1 and 8 to 11 or the nucleic acid encoding a Klotho protein for use of any one of claims 2 to 11, wherein the Klotho protein is a KL-VS variant.

13. The method of any one of claims 1 and 8 to 12 or the nucleic acid encoding a Klotho protein for use of any one of claims 2 to 12, wherein the nucleic acid does not integrate into the genome of the cell.

14. The method of any one of claims 1 and 8 to 13 or the nucleic acid encoding a Klotho protein for use of any one of claims 2 to 13, wherein the nucleic acid is administered in combination with an agent that increases endogenous Klotho expression.
